(19) Europäisches Patentamt
European Patent Office
Office européen des brevets

(11) EP 1 545 440 B1

(12) FASCICULE DE BREVET EUROPEEN

(45) Date de publication et mention de la délivrance du brevet:
**17.11.2010 Bulletin 2010/46**

(21) Numéro de dépôt: **03782505.6**

(22) Date de dépôt: **26.09.2003**

(51) Int Cl.:
*A61Q 1/06* (2006.01) *A61Q 1/04* (2006.01)
*A61K 8/90* (2006.01) *A61K 8/81* (2006.01)

(86) Numéro de dépôt international:
**PCT/FR2003/002844**

(87) Numéro de publication internationale:
**WO 2004/028490 (08.04.2004 Gazette 2004/15)**

(54) **COMPOSITION COSMETIQUE COMPRENANT UN POLYMERE SEQUENCE ET UN PLASTIFIANT**

KOSMETISCHE ZUSAMMENSETZUNG, DIE EINEN BLOCKPOLYMER UND EINEN WEICHMACHER ENTHÄLT

COSMETIC COMPOSITION COMPRISING A SEQUENCED POLYMER AND A PLASTICIZER

(84) Etats contractants désignés:
**AT BE BG CH CY CZ DE DK EE ES FI FR GB GR HU IE IT LI LU MC NL PT RO SE SI SK TR**

(30) Priorité: **26.09.2002 FR 0211949**
**20.12.2002 FR 0216437**
**21.05.2003 FR 0306121**

(43) Date de publication de la demande:
**29.06.2005 Bulletin 2005/26**

(73) Titulaire: **L'Oréal**
**75008 Paris (FR)**

(72) Inventeurs:
- **BLIN, Xavier**
**F-75015 Paris (FR)**
- **FERRARI, Véronique**
**F-94700 Maisons-Alfort (FR)**

(74) Mandataire: **Kromer, Christophe et al**
**L'Oréal**
**D.I.P.I.**
**25-29 Quai Aulagnier**
**92600 Asnières (FR)**

(56) Documents cités:

| | |
|---|---|
| EP-A- 1 159 950 | WO-A-00/26285 |
| DE-A- 10 029 697 | FR-A- 2 775 593 |
| FR-A- 2 798 061 | FR-A- 2 809 306 |
| GB-A- 1 324 745 | US-A- 6 153 206 |

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet une composition cosmétique comprenant un polymère séquencé et un plastifiant particuliers destinée à être appliquée sur les matières kératiniques d'êtres humains, comme la peau, les lèvres, les cils, les sourcils, les ongles, les cheveux. La composition est plus particulièrement destinée à être appliquée sur la peau ou les lèvres.

La composition selon l'invention peut être une composition de maquillage ou une composition de soin des matières kératiniques, notamment de la peau et des lèvres, et de préférence une composition de maquillage.

La composition de maquillage peut être un produit de maquillage des lèvres (rouge à lèvres), un fond de teint, un fard à paupières, un fard à joue, un produit anticernes, un eye-liner, un produit de maquillage du corps, un mascara, un vernis à ongles, un produit de maquillage des cheveux.

La composition de soin peut être un produit de soin de la peau du corps et du visage, notamment un produit solaire, un produit de coloration de la peau (tel qu'un autobronzant). La composition peut être également un produit capillaire, notamment pour le maintien de la coiffure ou la mise en forme des cheveux.

Les compositions de rouge à lèvres et fond de teint sont couramment employées pour apporter une couleur esthétique aux lèvres ou à la peau, notamment au visage. Ces produits de maquillage contiennent généralement des phases grasses telles que des cires et des huiles, des pigments et/ou charges et éventuellement des additifs comme des actifs cosmétiques ou dermatologiques.

Ces compositions, lorsqu'elles sont appliquées sur la peau, présentent l'inconvénient de transférer, c'est-à-dire de se déposer au moins en partie, en laissant des traces, sur certains supports avec lesquels elles peuvent être mises en contact et notamment un verre, une tasse, une cigarette, un vêtement ou la peau. Il s'ensuit une tenue médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

On recherche donc des compositions de maquillage des matières kératiniques, notamment des lèvres et de la peau avantageusement « sans transfert » qui présentent l'avantage de former un dépôt de bonne tenue, notamment qui ne se dépose pas, au moins en partie, sur les supports avec lesquels elles sont mises en contact (verre, vêtements, cigarette, tissus).

Pour améliorer la tenue des produits de maquillage, il est connu d'utiliser des polymères filmogènes. Par exemple, les documents US-6074654 et WO02/067877 proposent d'employer des résines de silicones. Mais l'introduction de ces polymères filmogènes peut conduire à des films trop durs qui ont alors tendance à se craqueler au cours du temps. En particulier, ces films durs ne suivent pas correctement le mouvement de la peau ou des lèvres et présentent donc une mauvaise tenue sur la peau ou les lèvres, le film se désagrégeant rapidement.

Par ailleurs, un film trop dure déposé sur les lèvres ou la peau va provoquer une sensation de tiraillement, rendant le dépôt inconfortable pour l'utilisatrice.

La présente invention a donc pour but de fournir une composition cosmétique apte à former un film souple, capable notamment de suivre les mouvement des lèvres ou de la peau.

Le brevet US 6 513 206 décrit des compositions cosmétiques et notamment des rouges à lèvres comprenant des copolymères éthyléniques linéaires préparés par GTP (Group Transfer Polymerisation) en association avec du triisostéaryl citrate. Le brevet GB 1 324 745 concerne des compositions de maquillage de type émulsion eau dans huile ou huile dans eau comprenant des copolymères blocs bi-
ou tri-séquencés émulsionnant, ou encore des polymères statistiques et envisagent leur association avec du diisopropyl adipate permettant une meilleure hydratation de la peau. La demande de brevet WO 00/26285 décrit des compositions sous forme de gel comprenant un polymère tribloc, étoilé, radial ou multibloc, linéaires ou greffés, en association avec un ester. La demande FR-A-2798061 décrit une composition cosmétique comprenant une dispersion de particules de polymères stabilisées en surface par un copolymère séquencé.

L'invention a également pour but de fournir une composition cosmétique formant un dépôt sur les matières kératiniques, en particulier sur la peau ou les lèvres, confortable au cours du temps.

Les inventeurs ont découvert qu'il est possible d'obtenir une telle composition en utilisant un polymère séquencé particulier associé à un plastifiant particulier.

De façon plus précise, la présente invention a donc pour objet une composition cosmétique, notamment pour le maquillage ou le soin des matières kératiniques, en particulier de la peau ou des lèvres, comprenant un milieu liquide organique cosmétiquement acceptable, un polymère séquencé et un plastifiant, caractérisée par le fait que :

- le polymère séquencé est un polymère éthylénique linéaire filmogène contenant des première et deuxième séquences reliées entre elles par un segment intermédiaire qui est un polymère statistique comprenant au moins un monomère constitutif de la première séquence et au moins un monomère constitutif de la deuxième séquence,
  la première séquence du polymère étant choisie parmi :

- a) une séquence ayant une Tg supérieure ou égale à 40°C,
- b) une séquence ayant une Tg inférieure ou égale à 20°C,
- c) une séquence ayant une Tg comprise entre 20 et 40°C, et
  la deuxième séquence étant choisie dans une catégorie a), b) ou c) différente de la première séquence ; et ledit polymère séquencé ayant un indice de polydispersité I supérieur ou égal à 2,8,

- le plastifiant étant tel que décrit ci-après.

Avantageusement, le polymère séquencé est exempt de styrène.
Avantageusement encore, le polymère séquencé est non élastomère.
De préférence également, les première et seconde séquences sont incompatibles l'une avec l'autre.
La composition selon l'invention permet d'obtenir un dépôt, notamment un dépôt de maquillage, sur les matières kératiniques, notamment sur la peau ou les lèvres ayant une bonne brillance et de bonnes propriétés de sans transfert.
La composition selon l'invention permet également d'obtenir un dépôt sur les matières kératiniques, notamment sur la peau ou les lèvres ne présentant pas de sensation de dessèchement, de tiraillement : le dépôt ainsi obtenu est donc confortable au cours du temps pour l'utilisatrice.
L'invention a également pour objet un procédé de maquillage des matières kératiniques, notamment de la peau ou des lèvres, comprenant l'application sur les matières kératiniques, notamment sur la peau ou les lèvres d'une composition, telle que définie précédemment.
L'invention a aussi pour objet l'utilisation d'une composition telle que définie précédemment pour obtenir un dépôt, notamment un maquillage, sur les matières kératiniques, en particulier sur la peau ou les lèvres, souple et/ou confortable au cours du temps.
Par milieu liquide organique cosmétiquement acceptable, on entend un milieu comprenant au moins un composé organique liquide à température ambiante (25 °C) et pression atmosphérique ($10^5$ Pa) compatible avec les matières kératiniques, notamment la peau, les lèvres, telles que les huiles ou les solvants organiques couramment employés dans les compositions cosmétiques. Le polymère séquencé de la composition selon l'invention est un polymère éthylénique séquencé linéaire filmogène.
Par polymère "éthylénique" , on entend un polymère obtenu par polymérisation de monomères comprenant une insaturation éthylénique.
Par polymère "séquencé", on entend un polymère comprenant au moins 2 séquences distinctes, de préférence au moins 3 séquences distinctes.
Le polymère est un polymère à structure linéaire. Par opposition, un polymère a structure non linéaire est, par exemple, un polymère à structure ramifiée, en étoile, greffée, ou autre. Par polymère "filmogène", on entend un polymère apte à former à lui seul ou en présence d'un agent auxiliaire de filmification, un film continu et adhérent sur un support, notamment sur les matières kératiniques.
Par "séquences incompatibles l'une avec l'autre", on entend que le mélange formé du polymère correspondant à la première séquence et du polymère correspondant à la deuxième séquence, n'est pas miscible dans le liquide organique majoritaire en poids du milieu liquide organique de la composition, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé +/- 15%.

Dans le cas où le milieu liquide organique comprend un mélange de liquide organiques, et dans l'hypothèse de deux ou plusieurs liquides organiques présents en proportions massiques identiques, ledit mélange de polymères est non miscible dans au moins l'un d'entre eux.
Bien entendu, dans le cas où le milieu liquide organique comprend un unique liquide organique, ce dernier est le liquide organique majoritaire.
Avantageusement, le liquide organique majoritaire de la composition est le solvant organique de polymérisation du polymère séquencé ou le solvant organique majoritaire du mélanges de solvant organiques de polymérisation du polymère séquencé.
La séquence intermédiaire est une séquence comprenant au moins un monomère constitutif de la première séquence et au moins un monomère constitutif de la deuxième séquence du polymère permet de "compatibiliser" ces séquences.
De façon préférentielle, le polymère utilisé dans la composition selon l'invention ne comprend pas d'atomes

de silicium dans son squelette. Par "squelette", on entend la chaîne principale du polymère, par opposition aux chaînes latérales pendantes.

De préférence, le polymère utilisé dans la composition selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25°C).

De préférence, le polymère utilisé dans la composition selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50 %.

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.
On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage. Les éprouvettes sont étirées à une vitesse de 50 mm/min et la distance entre les mors est de 50 mm, ce qui correspond à la longueur initiale ($I_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($I_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après:

$$R_i = (\varepsilon_{max} - \varepsilon_i) / \varepsilon_{max}) \times 100$$

Pour déterminer la recouvrance retardée, on mesure l'allongement résiduel de l'éprouvette en pourcentage ($\varepsilon_{2h}$).

La recouvrance retardée en % ($R_{2h}$) est donnée par la formule ci-après:

$$R_{2h} = (\varepsilon_{max} - \varepsilon_{2h}) / \varepsilon_{max}) \times 100$$

A titre purement indicatif, un polymère utilisé selon un mode de réalisation de l'invention possède une recouvrance instantanée $R_i$ de 10% et une recouvrance retardée $R_{2h}$ de 30%.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère utilisé dans la composition selon l'invention est de préférence inférieure ou égale à 300 000, elle va par exemple de 35 000 à 200 000, et mieux de 45 000 à 150 000.

La masse moyenne en nombre (Mn) du polymère utilisé dans la composition selon l'invention est de préférence inférieure ou égale à 70 000, elle va par exemple de 10 000 à 60 000, et mieux de 12 000 à 50 000.

L'indice de polydispersité du polymère utilisé dans la composition selon l'invention va notamment de 2,8 à 6.

Chaque séquence ou bloc du polymère utilisé dans la composition selon l'invention est issue d'un type de

monomère ou de plusieurs types de monomères différents.

Cela signifie que chaque séquence peut être constituée d'un homopolymère ou d'un copolymère ; ce copolymère constituant la séquence pouvant être à son tour statistique ou alterné.

De préférence, la séquence intermédiaire est issue essentiellement de monomères constitutifs de la première séquence et de la deuxième séquence. Par "essentiellement", on entend au moins à 85%, de préférence au moins à 90%, mieux à 95% et encore mieux à 100%.

Avantageusement, la séquence intermédiaire a une température de transition vitreuse Tg comprise entre les températures de transition vitreuse des première et deuxième séquences.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_{i} (\varpi_i / Tg_i) ,$$

$\omega_i$ étant la fraction massique du monomère i dans la séquence considerée et $Tg_i$ étant la température de transition vitreuse de l'homopolymère du monomère i.

Sauf indication contraire, les Tg indiquées pour les première et deuxième séquences dans la présente demande sont des Tg théoriques.

Avantageusement, les première et deuxième séquences du polymère sont telles que décrites précédemment.

On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

<u>a) Séquence avant une Tg supérieure ou égale à 40°C</u>

La séquence ayant une Tg supérieure ou égale à 40°C a par exemple une Tg allant de 40 à 150°C, de préférence supérieure ou égale à 50°C, allant par exemple de 50°C à 120°C, et mieux supérieure ou égale à 60°C, allant par exemple de 60°C à 120°C .

La séquence ayant une Tg supérieure ou égale à 40°C peut être un homopolymère ou un copolymère.

Dans le cas où cette séquence est un homopolymère, elle est issue de monomère dont l'homopolymère a une température de transition vitreuse supérieure ou égale à 40°C.

Dans le cas où cette séquence est un homopolymère, elle est issue de monomère dont l'homopolymère a une température de transition vitreuse supérieure ou égale à 40°C.

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères dont l'homopolymère a une Tg supérieure ou égale à 40°C, par exemple une Tg allant de 40 à 150°C, de préférence supérieure ou égale à 50°C, allant par exemple de 50°C à 120°C, et mieux supérieure ou égale à 60°C, allant par exemple de 60°C à 120°C, et
- des monomères dont l'homopolymère a une Tg inférieures à 40°C, choisis parmi les monomères dont l'homopolymère a une Tg comprise entre 20 à 40°C et/ou les monomères dont l'homopolymère a une Tg inférieure ou égale à 20°C, par exemple une Tg allant de -100 à 20°C, de préférence inférieure à 15°C, notamment allant de - 80°C à 15°C et mieux inférieure à 10°C, par exemple allant de -50°C à 0°C, tels que décrits plus loin, .

Les monomères dont l'homopolymère a une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$

dans laquelle $R_1$ représente un groupe alkyle non substitué, linéaire ou ramifié, contenant de 1 à 4 atomes de carbone, tel qu'un groupe méthyle, éthyle, propyle
ou isobutyle ou $R_1$ représente un groupe cycloalkyle $C_4$ à $C_{12}$,

- les acrylates de formule $CH_2 = CH-COOR_2$

dans laquelle $R_2$ représente un groupe cycloalkyle en $C_4$ à $C_{12}$ tel que un groupe isobornyle ou un groupe tertio butyle,

- les (méth)acrylamides de formule :

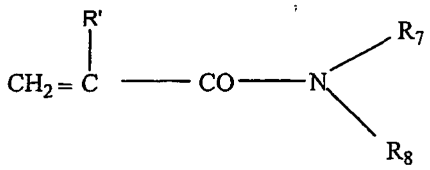

où $R_7$ et $R_8$ identiques ou différents représentent chacun un atome d'hydrogène
ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,

- et leurs mélanges.
  Des monomères principaux particulièrement préférés sont le méthacrylate de méthyle, le (méth)acrylate d'isobutyle, le (méth)acrylate d'isobornyle et leurs mélanges.

b) Séquence ayant une Tg inférieure ou égale à 20°C

La séquence ayant une Tg inférieure ou égale à 20°C a par exemple une Tg allant de -100 à 20°C, de préférence inférieure ou égale à 15°C, notamment allant de - 80°C à 15°C et mieux inférieure ou égale à 10°C, par exemple allant de -50°C à 0°C.
La séquence ayant une Tg inférieure ou égale à 20°C peut être un homopolymère ou un copolymère.
Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).
Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.
Elle peut par exemple comprendre

- un ou plusieurs monomères dont l'homopolymère correspondant a une Tg inférieure ou égale à 20°C, par exemple une Tg allant de -100°C à 20 °C, de préférence inférieure à 15°C, notamment allant de - 80°C à 15°C et mieux inférieur à 10°C, par exemple allant de -50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.
Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2 = C(CH_3)-COOR_4$,
  $R_4$ représentant un groupe alkyle non substitué en $C_6$ à $C_{12}$ linéaire ou ramifié,

dans lequel se trouve(nt) éventuellement intercalé(s) un ou plusieurs hétéroatomes choisis parmi 0, N et S;

- les esters de vinyle de formule $R_5$-CO-O-CH = $CH_2$

où $R_5$ représente un groupe alkyle en $C_4$ à $C_{12}$ linéaire ou ramifié ;

- les éthers de vinyle et d'alkyle en $C_4$ à $C_{12}$, tels que l'éther de vinyle et de méthyle et l'éther de vinyle et d'éthyle.
- les N-alkyl en $C_4$ à $C_{12}$ acrylamides, tels que le N-octylacrylamide,
- et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

c) Séquence ayant une Tg comprise entre 20 et 40°C

La séquence qui a une Tg comprise entre 20 et 40°C peut être un homopolymère ou un copolymère.
Dans le cas où cette séquence est un homopolymère, elle est issue de monomère (ou monomère principal) dont l'homopolymère a une température de transition vitreuse comprises entre 20 et 40°C.
Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.
Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40°C.
Avantageusement, la séquence ayant une Tg comprise entre 20 et 40°C est un copolymère issue en totalité ou en partie :

- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C, par exemple allant de -50°C à 0°C, tels que décrits plus haut,
  lesdits monomères étant choisis de telle sorte que la Tg du copolymère formant la première séquence est comprise entre 20 et 40°C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.
De préférence, la proportion de la deuxième séquence ayant une Tg inférieure ou égale à 20°C va de 10 à 85% en poids du polymère, mieux de 20 à 70% et encore mieux de 20 à 50%.
Selon un mode de réalisation, le polymère séquencé utilisé dans la composition selon l'invention est exempt de styrène. Par polymère exempt de styrène, on entend un polymère comprenant moins de 10%, de préférence moins de 5%, de préférence moins de 2%, de préférence encore moins de 1 % en poids, voire ne contient pas, de monomère styrénique tels que le styrène ou les dérivés du styrène comme par exemple le méthylstyrène, le chlorostyrène ou le chlorométhylstyrène.
Selon un mode de réalisation, le polymère séquencé de la composition selon l'invention est issu de monomères éthyléniques aliphatiques. Par monomère aliphatique, on entend un monomère ne comprenant aucun groupe aromatique.
Chacune des séquences peut néanmoins contenir en proportion minoritaire au moins un monomère constitutif de l'autre séquence. Ainsi la première séquence peut contenir au moins un monomère constitutif de la deuxième séquence et inversement.
Chacune des première et/ou deuxième séquence, peu(ven)t comprendre, outre les monomères indiqués ci-dessus, un ou plusieurs autres monomères appelés monomères additionnels, différents des monomères principaux cités précédemment. La nature et la quantité de ce ou ces monomères additionnels sont choisies de manière à ce que la séquence dans laquelle ils se trouvent ait la température de transition vitreuse désirée.
Ce monomère additionnel est par exemple choisi parmi :

les monomères hydrophiles tels que :

- les monomères à insaturation(s) éthylénique(s) comprenant au moins une fonction acide carboxylique ou sulfonique comme par exemple : l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)\text{-}COOR_6$ dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2 = C(CH_3)\text{-}COOR_9$, $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$, $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris ( triméthylsiloxy) silane,

- et leurs mélanges.

Des monomères additionnels particulièrement préférés sont l'acide acrylique, l'acide méthacrylique, le méthacrylate de trifluoroéthyle et leurs mélanges.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

Selon un mode préféré de réalisation, le polymère utilisé dans la composition selon l'invention est un polymère non siliconé, c'est à dire un polymère exempt d'atome de silicium.

De préférence, chacune des première et deuxième séquences comprend au moins un monomère choisi parmi les esters d'acide (méth)acrylique tels que définis précédemment, et éventuellement un monomère choisi parmi l'acide (méth)acrylique, et leurs mélanges. Avantageusement, chacune des première et deuxième séquences est issue en totalité d'au moins un monomère choisi parmi les esters d'acide (méth)acrylique tels que définis précédemment, et éventuellement un monomère choisi parmi l'acide (méth)acrylique, et leurs mélanges.

Le polymère utilisé dans la composition selon l'invention peut être obtenu par polymérisation radicalaire en solution selon le procédé de préparation suivant :

une partie du solvant de polymérisation est introduite dans un réacteur adapté et chauffée jusqu'à atteindre la température adéquate pour la polymérisation (typiquement entre 60 et 120°C),

une fois cette température atteinte, les monomères constitutifs de la première séquence sont introduits en présence d'une partie de l'initiateur de polymérisation, au bout d'un temps T correspondant à un taux de conversion maximum de 90%, les monomères constitutifs de la deuxième séquence et l'autre partie de l'initiateur sont introduits,

on laisse réagir le mélange pendant un temps T' ( allant de 3 à 6 h) au bout duquel le mélange est ramené à température ambiante,

on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un

mélange acétate de butyle et isopropanol ou l'isododécane.

Selon un premier mode de réalisation, le polymère utilisé dans la composition selon l'invention comprend au moins une (notamment une) première séquence ayant une Tg supérieure ou égale à 40°C, telle que décrite plus haut au a) et au moins une (notamment une) deuxième séquence ayant une Tg inférieure ou égale à 20°C, telle que décrite plus haut au b).

De préférence, la première séquence ayant une Tg supérieure ou égale à 40°C est un copolymère issu de monomères dont l'homopolymère a une température de transition vitreuse supérieure ou égale à 40°C, tels que les monomère décrits précédemment. Avantageusement, la deuxième séquence ayant une Tg inférieure ou égale à 20°C est un homopolymère, notamment issu de monomère tels que décrits précédemment.

De préférence, la proportion de la séquence ayant une Tg supérieure ou égale à 40°C va de 20 à 90% en poids du polymère, mieux de 30 à 80% et encore mieux de 50 à 70%. De préférence, la proportion de la séquence ayant une Tg inférieure ou égale à 20°C va de 5 à 75% en poids du polymère, de préférence de 15 à 50% et mieux de 25 à 45%.

Ainsi, selon une première variante, le polymère utilisé dans la composition selon l'invention peut comprendre :

- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante, le polymère utilisé dans la composition selon l'invention peut comprendre :

- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et
- une séquence intermédiaire qui est un copolymère statistique méthacrylate de méthyl/acide acrylique/acrylate de méthyle/méthacrylate de trifluoroéthyle.

Selon une troisième variante, le polymère utilisé dans la composition selon l'invention peut comprendre :

- une première séquence de Tg supérieure ou égale à 40°C, par exemple allant de 85 à 115°C, qui est un copolymère acrylate d'isobornyle/méthacrylate d'isobutyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon une quatrième variante, le polymère utilisé dans la composition selon l'invention peut comprendre :

- une première séquence de Tg supérieure ou égale à 40°C, par exemple allant de 85 à 115°C, qui est un copolymère acrylate d'isobornyle/méthacrylate de méthyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate de méthyle/acrylate d'éthyl-2 hexyle.

Selon une cinquième variante, le polymère utilisé dans la composition selon l'invention peut comprendre :

- une première séquence de Tg supérieure ou égale à 40°C, par exemple allant de 95 à 125°C, qui est un copolymère acrylate d'isobornyle / méthacrylate d'isobornyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/ méthacrylate d'isobornyle/ acrylate d'éthyl-2 hexyle.

Selon une sixième variante, le polymère utilisé dans la composition selon l'invention peut comprendre :

- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobutyle/ acrylate d'isobutyle.

Selon une septième variante, le polymère utilisé dans la composition selon l'invention peut comprendre :

- une première séquence de Tg supérieure ou égale à 40°C, par exemple allant de 95 à 125°C, qui est un copolymère acrylate d'isobornyle/méthacrylate d'isobornyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobornyle/ acrylate d'isobutyle.

Selon une huitième variante, le polymère utilisé dans la composition selon l'invention peut comprendre :

- une première séquence de Tg supérieure ou égale à 40°C, par exemple allant de 60 à 90°C, qui est un copolymère acrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique acrylate d'isobornyle/méthacrylate d'isobutyle/acrylate d'isobutyle.

Selon un second mode de réalisation, le polymère utilisé dans la composition selon l'invention comprend au moins une (notamment une) première séquence ayant une température de transition vitreuse (Tg) comprise entre 20 et 40°C, conforme aux séquences décrites au c) et au moins une (notamment une) deuxième séquence ayant une température de transition vitreuse inférieure ou égale à 20°C, telle que décrite plus haut au b) ou une température de transition vitreuse supérieure ou égale à 40°C, telle que décrite au a) ci-dessus.

De préférence, la proportion de la première séquence ayant une Tg comprise entre 20 et 40°C va de 10 à 85% en poids du polymère, mieux de 30 à 80% et encore mieux de 50 à 70%.

Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.

Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.

De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

Avantageusement, la deuxième séquence ayant une Tg inférieure ou égale à 20°C ou ayant une Tg supérieure ou égale à 40°C est un homopolymère.

Ainsi, selon première variante de ce second mode de réalisation, le polymère utilisé dans la composition selon l'invention peut comprendre :

- une première séquence de Tg comprise entre 20 et 40°C, par exemple ayant une Tg de 25 à 39°C, qui est un copolymère comprenant au moins un monomère acrylate de méthyle, au moins un monomère méthacrylate de méthyle et au moins un monomère acide acrylique,
- une deuxième séquence de Tg supérieure ou égale à 40°C, par exemple allant de 85 à 125°C, qui est un homopolymère composé de monomères méthacrylate de méthyle et
- une séquence intermédiaire comprenant au moins un monomère acrylate de méthyle, méthacrylate de méthyle et
- une séquence intermédiaire comprenant au méthacrylate de méthyle, au moins un monomère acide acrylique et au moins un monomère acrylate de méthyle.

Selon seconde variante de ce second mode de réalisation, le polymère utilisé dans la composition selon l'invention peut comprendre :

- une première séquence de Tg comprise entre 20 et 40°C, par exemple ayant une Tg de 21 à 39°C, qui est un

copolymère comprenant acrylate d'isobornyle/méthacrylate d'isobutyle/acrylate d'éthyl-2 hexyle,

- une deuxième séquence de Tg inférieure ou égale à 20°C, par exemple allant de -65 à -35°C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une troisième variante de ce second mode de réalisation, le polymère utilisé dans la composition selon l'invention peut comprendre :

- une première séquence de Tg comprise entre 20 et 40°C, par exemple ayant une Tg de 21 à 39°C, qui est un copolymère acrylate d'isobornyle/acrylate de méthyle/acide acrylique,
- une deuxième séquence de Tg isupérieure ou égale à 40°C, par exemple allant de 85 à 115°C, qui est un homopolymère d'acrylate d'isobornyle et
- une séquence intermédiaire qui est un copolymère statistique acrylate d'isobornyle / acrylate de méthyle/acide acrylique.

Le polymère séquencé décrit précédemment peut être présent dans la composition selon l'invention en une teneur allant de 0,1 % à 90 % en poids, par rapport au poids total de la composition, de préférence allant de 0,5 % à 50 % en poids, et préférentiellement allant de 0,5 % à 30 % en poids.

Le plastifiant présent dans la composition selon l'invention peut être un composé dont la nature et la quantité sont telles que la composition est apte à former un film ayant une dureté inférieure ou égale à 35 secondes, la dureté du film étant mesurée à l'aide d'un pendule de Persoz selon la norme NF-T-30-016.

Aussi, selon un mode de réalisation de l'invention, la composition comprend un polymère séquencé tel que décrit précédemment et un plastifiant dont la nature et la quantité sont telles que la composition est apte à former un film ayant une dureté inférieure ou égale à 35 secondes, la dureté du film étant mesurée à l'aide d'un pendule de Persoz selon la norme NF-T-30-016.

La dureté du film est mesurée pour une couche de 300 µm d'épaisseur (avant séchage), déposée sur une plaque de verre chauffée à 30 °C et après séchage, pendant 23 heures à atmosphère ambiante puis pendant 1 heure, à 70 % d'humidité relative pour une composition à milieu solvant ou à 50 % d'humidité relative pour une composition à milieu aqueux. La dureté du film obtenu est mesurée selon la norme ASTM D-43-66, ou la norme NF-T 30-016 (décembre 1991), à l'aide d'un pendule de Persoz.

Avantageusement, la composition selon l'invention est apte à former un film ayant une dureté inférieure ou égale à 35 secondes, notamment allant de 8 à 35 secondes, de préférence inférieure ou égale à 30 secondes, notamment allant de 10 à 30 secondes, et préférentiellement inférieure ou égale à 25 secondes, notamment allant de 12 à 25 secondes.

Le plastifiant présent dans la composition selon l'invention peut être un composé ayant un paramètre de solubilité $\delta_h$ allant de 5,5 à 11 $(J/cm^3)^{1/2}$.

Aussi, selon un mode de réalisation de l'invention, la composition comprend un polymère séquencé tel que décrit précédemment et un plastifiant qui est un composé ayant un paramètre de solubilité $\delta_h$ allant de 5,5 à 11 $(J/cm^3)^{1/2}$.

Le plastifiant présent dans la composition selon l'invention peut être un composé ayant un paramètre de solubilité $\delta_h$ allant de 5,5 à 11 $(J/cm^3)^{1/2}$, de préférence allant de 5,5 à 11, préférentiellement allant de 5,9 à 11, de préférence allant de 7 à 10,5, de préférence allant de 9 à 10, plus préférentiellement allant de 8 à 10 $(J/cm^3)^{1/2}$.

De préférence, le composé plastifiant a un paramètre de solubilité $\delta_P$ allant de 1,5 à 4,5 $(J/cm^3)^{1/2}$, de préférence allant de 1,5 à 4 $(J/cm^3)^{1/2}$, de préférence allant de 1,5 à 3,5 $(J/cm^3)^{1/2}$, de préférence encore allant de 2 à 3 $(J/cm^3)^{1/2}$.

Le plastifiant est de préférence liquide à température ambiante et pression atmosphérique.

La définition des paramètres de solubilité selon HANSEN est bien connue de l'homme du métier, et notamment décrite dans l'article de C. M. HANSEN : "The three dimensional solubility parameters" J. Paint Technol. 39, 105 (1967). Ces paramètres sont aussi décrits dans le document JP-A-08-109121 de KAO et le document de D.W. Van KREVELEN "Properties of polymers" (1990), p. 190.

Selon cet espace de HANSEN :

- $\delta_P$ caractérisé les forces d'interactions de DEBYE entre dipôles permanents ; et
- $\delta_h$ caractérise les forces d'interactions spécifiques (type liaisons hydrogène, acide/base, donneur/accepteur, etc.) ;

Les paramètres $\delta_P$ et $\delta_h$ sont généralement exprimés en $(J/cm^3)^{1/2}$. Ils sont déterminés à température ambiante (25°C) et en particulier selon la méthode de calcul indiquée dans le document brevet de KAO ci-dessus.

Dans la composition selon l'invention, on peut utiliser un mélange de composés satisfaisant aux relations ci-dessus. Dans ce cas, les paramètres de solubilité du mélange sont déterminés à partir de ceux des composés pris

séparément, selon les relations suivantes:

$$\delta_{pmel} = \sum_{i} xi\, \delta_{pi} \quad \text{et} \quad \delta_{hmel} = \sum_{i} xi\, \delta_{hi}$$

où xi représente la fraction volumique du composé (i) dans le mélange.

Il est à la portée de l'homme du métier de déterminer les quantités de chaque composé pour obtenir un mélange de composés répondant aux relations ci-dessus.

De préférence, le plastifiant a une masse moléculaire inférieure ou égale à 5000 g/mol, de préférence inférieure ou égale à 2000 g/mol, préférentiellement inférieure ou égale à 1000 g/mol, et plus préférentiellement inférieure ou égale à 900 g/mol. Le plastifiant a avantageusement une masse moléculaire supérieure ou égale à 100 g/mol.

Avantageusement, le plastifiant utilisé selon l'invention est un ester.

Selon un premier mode de réalisation de la composition selon l'invention, le plastifiant peut être choisi parmi les esters d'au moins un acide carboxylique comprenant 1 à 7 atomes de carbones et d'un polyol comprenant au moins 4 groupes hydroxyles.

Aussi, l'invention concerne une composition comprend un polymère séquencé tel que décrit précédemment et un plastifiant choisi parmi les esters d'au moins un acide carboxylique comprenant 1 à 7 atomes de carbones et d'un polyol comprenant au moins 4 groupes hydroxyles, ledit ester ayant une masse moléculaire inférieure à 5000 g/mol.

Le polyol selon l'invention peut être un ose - polyhydroxyaldéhyde (aldose) ou polyhydroxycétone (cétose) - cyclisé ou non. Le polyol est de préférence un ose cyclisé sous forme d'hémi-acétal.

Parmi les aldoses, on peut citer le D-ribose, le D-xylose, le L-arabinose, le D-glucose (ou alpha-D-glucopyranose lorsqu'il est sous forme d'hémi-acétal cyclique), le D-mannose, et le D-galactose.

Parmi les cétoses, on peut citer le D-xylulose et le D-fructose (ou béta-D-fructofuranose lorsqu'il est sous forme d'hémi-acétal cyclique).

Le polyol peut être un mono- ou un polysaccharide comprenant de 1 à 10 oses, de préférence de 1 à 4, de préférence encore un ou deux oses. Le polyol peut être choisi parmi l'érythritol, le xylitol, le sorbitol, le glucose, le saccharose, le lactose, le maltose.

Le polyol selon l'invention est de préférence un disaccharide. Parmi les disaccharides, on peut citer le saccharose (appelé également alpha-D-glucopyranosyl-(1-2)-béta-D-fructofuranose), le lactose (appelé également béta-D-galactopyranosyl-(1-4)-béta-D-glucopyranose) et le maltose (appelé également alpha-D-glucopyranosyl-(1-4)-béta-D-glucopyranose), et de préférence le saccharose.

Le polyol peut être un polysaccharide constitué de plusieurs oses identiques ou d'au moins deux oses différents.

L'ester selon l'invention peut être constitué d'un polyol estérifié par au moins deux acides monocarboxyliques différents, ou par au moins trois acides monocarboxyliques différents.

L'ester selon l'invention peut être un copolymère de deux esters, en particulier un copolymère i) d'un saccharose substitué par des groupements benzoyle et ii) d'un saccharose substitué par des groupements acétyle et/ou isobutyryle.

L'acide carboxylique est de préférence un acide monocarboxylique comprenant de 1 à 7 atomes de carbones, de préférence de 1 à 5 atomes de carbone, par exemple choisi parmi les acides acétique, n-propanoïque, isopropanoïque, n-butanoïque, isobutanoïque, tertiobutanoïque, n-pentanoïque et benzoïque.

L'ester peut être obtenu à partir d'au moins deux acides monocarboxyliques différents. Selon un mode de mise en oeuvre, l'acide est un acide linéaire ou ramifié non substitué.

L'acide est de préférence choisi parmi l'acide acétique, l'acide isobutyrique, l'acide benzoïque, et leurs mélanges , et plus préférentiellement.

Selon un mode de mise en oeuvre préféré, l'ester est le di-acétate hexa-(2-méthyl-propanoate) de saccharose, tel que celui vendu sous la dénomination "Sustane SAIB Food Grade Kosher" par la société EASTMAN CHEMICAL.

Selon un deuxième mode de réalisation de la composition selon l'invention, le plastifiant peut être choisi parmi les esters d'acide polycarboxylique aliphatique ou aromatique et d'alcool aliphatique ou aromatique comprenant de 1 à 10 atomes de carbone.

Aussi, l'invention concerne une composition comprend un polymère séquencé tel que décrit précédemment et un plastifiant choisi parmi les esters d'acide polycarboxylique aliphatique ou aromatique et d'alcool aliphatique ou aromatiques comprenant de 1 à 10 atomes de carbone.

L'alcool aliphatique ou aromatique comprend de 1 à 10 atomes de carbone, de préférence de 1 à 8, par exemple de 1 à 6. Il peut être choisis parmi les alcools R1OH, tels que R1 représente méthyle, éthyle, propyle, isopropyle, butyle, hexyle, éthylhexyle, décyle, isodécyle, benzyle, ou benzyle substitué par un alkyle comprenant 1 à 3 atomes de carbone, et leurs mélanges.

L'acide polycarboxylique aliphatique ou aromatique comprend de préférence de 3 à 12 atomes de carbone,

de préférence de 3 à 10 atomes de carbone, de préférence de 3 à 8 atomes de carbone, par exemple 6 ou 8 atomes de carbones.

L'acide polycarboxylique aliphatique ou aromatique est avantageusement choisi parmi les acides dicarboxyliques et les acides tricarboxyliques.

Parmi les acides dicarboxyliques aliphatiques, on peut citer ceux de formule HOOC-(CH2)n-COOH, dans laquelle n est un entier allant de 1 à 10, de préférence allant de 2 à 8, par exemple égal à 2, 4, 6 ou 8. On préfère les acides dicarboxyliques choisis parmi l'acide succinique, l'acide adipique et l'acide sébacique.

Parmi les acides dicarboxyliques aromatiques, on peut citer l'acide phtalique.

Parmi les acides tricarboxyliques, on peut citer les triacides qui correspondent à la formule

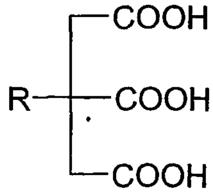

dans laquelle R représente un groupement -H, -OH ou -OCOR' dans lequel R' représente un groupement alkyle ayant de 1 à 6 atomes de carbone. De préférence, R représente un groupement $-OCOCH_3$.

L'acide tricarboxylique est notamment choisi parmi l'acide acétyl-citrique, l'acide butyroyl-citrique, l'acide citrique.

Parmi les esters d'acide tricarboxylique, on peut utiliser les esters dérivés de l'acide citrique ( ou citrates) tels que l'acétyl-citrate de tributyle, l'acétyl-citrate de triéthyle, l'acétyl-citrate de triéthylhexyle, l'acétyl-citrate de trihexyle, le butyroylcitrate de trihexyle, le citrate de triisodécyle, le citrate de triisopropyle, le citrate de tributyle et le citrate de tri (éthyl-2- hexyle).

Parmi les esters de l'acide adipique, on peut citer l'adipate de dibutyle et l'adipate de di(éthyl-2-hexyle).

Parmi les esters de l'acide sébacique, on peut citer le sébaçate de dibutyle, le sébaçate de di(éthyl-2-hexyle), le sébaçate de diéthyle et le sébaçate de diisopropyle.

Parmi les esters de l'acide succinique, on peut citer le succinate de di(éthyl-2-hexyle) et le succinate de diéthyle.

Parmi les esters de l'acide phtalique, on peut citer le phtalate de butyle et de benzyle, le phtalate de dibutyle, le phtalate de diéthylhexyle, le phtalate de diéthyle et le phtalate de diméthyle.

Le plastifiant ne comprend de préférence aucun groupe polaire à l'exception du groupe ester, et en particulier ne comprend aucun groupe hydroxyle. Les "groupes polaires" sont par exemple des groupes polaires ioniques ou non ioniques choisis parmi -COOH ; -OH ; oxyde d'éthylène; oxyde de propylène; $-PO_4$ ; -NHR ; $-NR_1R_2$ avec $R_1$, $R_2$ représentant un radical alkyle ou alkoxy en $C_1$ à $C_{20}$. , pouvant être linéaire, ramifié ou cyclique.

Avantageusement, le polymère séquencé et le plastifiant peuvent être présents dans la composition en une teneur telle que le rapport massique entre le polymère séquencé et le plastifiant est compris entre 0,5 et 100, de préférence entre 1 et 50, de préférence entre 1 et 10, de préférence encore entre 1 à 5.

Selon un mode de réalisation particulièrement préféré, le milieu liquide organique de la composition contient au moins un liquide organique qui est le ou un des solvants organique(s) de polymérisation du polymère séquencé tel que décrit précédemment. Avantageusement, ledit solvant organique de polymérisation est le liquide organique majoritaire en poids dans le milieu liquide organique de la composition cosmétique.

La composition selon l'invention peut comprendre en outre au moins une huile volatile.

Par "huile", on entend tout milieu non aqueux liquide à température ambiante (25°C) et pression atmosphérique (760mm de Hg), compatible avec une application sur la peau, les muqueuses (lèvres) et/ou les phanères (ongles, cils, sourcils, cheveux).

Par huile volatile, on entend tout milieu non aqueux susceptible de s'évaporer de la peau ou des lèvres, en moins d'une heure, ayant notamment une pression de vapeur, à température ambiante et pression atmosphérique allant de $10^{-3}$ à 300 mm de Hg (0,13 Pa à 40 000 Pa).

Selon l'invention, on peut utiliser une ou plusieurs huiles volatiles.

Ces huiles peuvent être des huiles hydrocarbonées ou des huiles siliconées comportant éventuellement des groupements alkyle ou alkoxy pendants ou en bout de chaîne siliconée.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Comme huile de silicone volatile utilisable dans l'invention, on peut citer les silicones linéaires ou cycliques

ayant de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme autre huile volatile utilisable dans l'invention, on préfère notamment les isoparaffines en $C_8$-$C_{16}$ comme l'isododécane, l'isodécane, l'isohexadécane et par exemple les huiles vendues sous les noms commerciaux d'ISOPAR, de PERMETHYL et notamment l'isododécane (PERMETHYL 99 A).

L'huile volatile peut être présente dans la composition selon l'invention en une teneur allant de 0,1 % à 90 % en poids, par rapport au poids total de la composition, de préférence allant de 1 % à 70 % en poids, et préférentiellement allant de 5 % à 50 % en poids.

La composition selon l'invention peut comprendre une huile non volatile.

On entend par huile non volatile une huile susceptible de rester sur la peau à température ambiante (25 °C) et pression atmosphérique au moins une heure et ayant notamment une pression de vapeur à température ambiante (25 °C) et pression atmosphérique, non nulle, inférieure à 0,01 mm de Hg (1,33 Pa).

L'huile non volatile peut être choisie parmi les huiles non volatiles siliconées ou hydrocarbonées.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

L'huile non volatile peut être choisie parmi les huiles non volatiles apolaires, les huiles non volatiles polaires, et leurs mélanges.

L'huile non volatile peut être présente dans la composition selon l'invention en une teneur allant de 0,1 % à 20 % en poids, par rapport au poids total de la composition, de préférence allant de 1 % à 15 % en poids, et préférentiellement allant de 1 % à 10 % en poids.

Comme huile non volatile utilisable dans l'invention, on peut citer :

- les huiles non volatile hydrocarbonées telles que l'huile de paraffine ( ou vaseline), le squalane, le polyisobutylène hydrogéné (huile de Parléam), le perhydrosqualène, l'huile de vison, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, notamment en $C_{12}$-$C_{36}$, tels que, le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine ; les acides gras supérieurs, notamment en $C_{14}$-$C_{22}$, tels que l'acide myristique, l'acide palmitique, l'acide stéarique, l'acide béhénique, l'acide oléique, l'acide linoléique, l'acide linolénique ou l'acide isostéarique ; les alcools gras supérieurs, notamment en $C_{16}$-$C_{22}$, tels que le cétanol, l'alcool oléique, l'alcool linoléique ou linolénique, l'alcool isostéarique ou l'octyl dodécanol ; et leurs mélanges.
- les huiles siliconées non volatiles telles que les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes ; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène) ; les silicones aminées ; les silicones à groupement hydroxyles ; les silicones fluorés comportant un groupement fluoré pendant ou en bout de chaîne siliconée ayant de 1 à 12 atomes de carbone dont tout ou partie des hydrogène sont substitués par des atomes de fluor ; et leurs mélanges

Avantageusement, l'huile non volatile hydrocarbonée est choisie parmi les hydrocarbures notamment les alcanes comme le polyisobutène hydrogéné.

La composition peut comprendre, outre le polymère séquencé décrit précédemment selon l'invention, un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

La composition selon l'invention peut également comprendre au moins corps gras solides à température ambiante notamment choisi parmi les cires, les corps gras pâteux, les gommes et leurs mélanges. Ces corps gras peuvent être d'origine animale, végétale, minérale ou synthétique.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120°C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,1 à 50 % en poids de cires, par rapport au poids total de la composition et mieux de 1 à 30 % en poids.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition. Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple

le stéarate de zinc, de magnésium ou de lithium, le laurate de zinc, le myristate de magnésium.

La composition selon l'invention peut également contenir des ingrédients couramment utilisés en cosmétique, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents anti-pelliculaires, les agents propulseurs, ou leurs mélanges.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 $s^{-1}$, après 10 minutes de mesure en géométrie cône/plan). La composition peut être anhydre, par exemple il peut s'agir d'un stick ou d'une pâte anhydre. La composition peut être une composition non rincée.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Selon un autre aspect, l'invention concerne également un ensemble cosmétique comprenant :

i) un récipient délimitant au moins un compartiment, ledit récipient étant fermé par un élément de fermeture ; et
ii) une composition disposée à l'intérieur dudit compartiment, la composition étant conforme à l'inevention.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une

tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

Le contenu des brevets ou demandes de brevets cités précédemment sont incorporés par référence dans la présente demande.

L'invention est illustrée plus en détails par les exemples décrits ci-après.

**Exemple 1 :**

**Préparation d'un polymère de poly(acrylate d'isobornyle/méthacrylate d'isobornyle /acrylate d'éthyl-2 hexyle)**

100 g d'isododécane sont introduits dans un réacteur de 1 litre, puis on augmente la température de façon à passer de la température ambiante (25°C) à 90°C en 1 heure.

On ajoute ensuite, à 90°C et en 1 heure, 105 g d'acrylate d'isobornyle, 105 g de méthacrylate d'isobomyl, 110 g d'isododécane et 1,8 g de 2.5- Bis(2-éthylhexanoylperoxy)-2.5-diméthylhexane (Trigonox® 141 d'Akzo Nobel).
Le mélange est maintenu 1 h 30 à 90°C.
On introduit ensuite au mélange précédent, toujours à 90°C et en 30 minutes, 90 g d'acrylate d'éthyle-2 hexyle, 90 g d'isododécane et 1,2 g de 2.5- Bis(2-éthylhexanoylperoxy)-2.5-diméthylhexane.
Le mélange est maintenu 3 heures à 90°C, puis l'ensemble est refroidi.
On obtient une solution à 50% de matière active en polymère dans l'isododécane.

On obtient un polymère comprenant une première séquence ou bloc poly(acrylate d'isobornyle/méthacrylate d'isobornyle) ayant une Tg de 110°C, une deuxième séquence polyacrylate d'éthyl-2 hexyle ayant une Tg de - 70°C et une séquence intermédiaire qui est un polymère statistique acrylate d'isobornyle/méthacrylate d'isobornyle/acrylate d'éthyl-2 hexyle.

Ce polymère présente une masse moyenne en poids de 103 900 et une masse moyenne en nombre de 21 300, soit un indice de polydispersité I de 4.89.

**Exemple 2 :**

On a préparé un rouge à lèvres ayant la composition suivante :

| | | |
|---|---|---|
| Polymère séquencé de l'exemple 1 à 50 % en poids dans l'isododécane | | 50 g |
| Silice pyrogénée traitée hydrophobe (Aérosil R 972 de Degussa) | | 5 g |
| Acétate isobutyrate de saccharose (EASTMAN SAIB d' EASTMAN CHEMICAL) | | 5 g |
| Polyisobutylène hydrogéné (huile de Parleam) | | 2,1 g |
| Octyldodécanol | | 0,9 g |
| Huile de silicone phénylée (Dow Corning 556 C) | | 2,1 g |
| Copolymère de polyvinyl pyrrolidone/Eicosène (Antaron V220 d'ISP) | | 1,2 g |
| Pigments | | 3 g |
| Isododécane | qsp | 100 g |

On mélange l'octydodécanol, l'huile siliconée, l'huile de Parléam, l'acétate isobutyrate de saccharose, et le copolymère de polyvinyl pyrrolidone/Eicosène en chauffant à environ 60°C. On réalise avec ce mélange un broyat pigmentaire des pigments en effectuant 3 passages du mélange à la broyeuse tri cylindres.

On mélange ensuite à température ambiante le broyat pigmentaire, l'isododécane, le polymère séquencé, puis on introduit au final la silice. La formule est ensuite introduite dans une bouillote étanche.

Le rouge à lèvres forme un film ayant une dureté de 20 secondes, mesurée au pendule de Persoz selon le

protocole décrit précédemment.

Ce rouge à lèvres permet donc d'obtenir un maquillage souple, confortable à porter dans le temps , et présentant un bon non transfert.

**Exemple 3 :**

On a préparé un rouge à lèvres ne contenant pas de plastifiant, ne faisant donc pas partie de l'invention, ayant la composition suivante :

| | | |
|---|---|---|
| Polymère séquencé de l'exemple 1 à 50 % en poids dans l'isododécane | | 50 g |
| Silice pyrogénée traitée hydrophobe (Aérosil R 972 de Degussa) | | 5 g |
| Polyisobutylène hydrogéné (huile de Parleam) Octyldodécanol | | 2,1 g 0,9 g |
| Huile de silicone phénylée (Dow Corning 556 C) | | 2,1 g. |
| Copolymère de polyvinyl pyrrolidone/Eicosène (Antaron V220 d'ISP) | | 1,2 g |
| Pigments | | 3 g |
| Isododécane | qsp | 100 g |

Ce rouge à lèvres forme un film ayant une dureté de 42 secondes. Il forme donc un maquillage moins souple que celui de obtenu avec le rouge à lèvres de l'exemple 2 selon l'invention.

**Revendications**

1. Composition cosmétique comprenant un milieu liquide organique cosmétiquement acceptable, un polymère séquencé et un plastifiant, **caractérisée par le fait que** :

   - le polymère séquencé est un polymère éthylénique linéaire filmogène contenant des première et deuxième séquences reliées entre elles par un segment intermédiaire qui est un polymère statistique comprenant au moins un monomère constitutif de la première séquence et au moins un monomère constitutif de la deuxième séquence,
   la première séquence du polymère étant choisie parmi :

     - a) une séquence ayant une Tg supérieure ou égale à 40°C,
     - b) une séquence ayant une Tg inférieure ou égale à 20°C,
     - c) une séquence ayant une Tg comprise entre 20 et 40°C, et

   la deuxième séquence étant choisie dans une catégorie a), b) ou c) différente de la première séquence ;
   et ledit polymère séquencé ayant un indice de polydispersité I supérieur ou égal à 2,8,
   - le plastifiant étant un composé dont la nature et la quantité sont telles que la composition est apte à former un film ayant une dureté inférieure ou égale à 35 secondes, la dureté du film étant mesurée à l'aide d'un pendule de Persoz selon la norme NF-T-30-016.

2. Composition selon la revendication 1, **caractérisée par le fait que** le film a une dureté inférieure à 30 secondes, de préférence inférieure à 25 secondes.

3. Composition selon la revendication 1, **caractérisée par le fait que** le film a une dureté allant de 8 à 35 secondes, de préférence allant de 10 à 30 secondes, et préférentiellement allant de 12 à 25 secondes.

4. Composition selon l'une quelconque des revendications précédentes, **caractérisée par le fait que** le plastifiant est un composé ayant un paramètre de solubilité $\delta_h$ allant de 5,5 à 11 $(J/cm^3)^{1/2}$.

5. Composition selon l'une quelconque des revendications qui précèdent **caractérisée en ce que** le polymère séquencé est exempt de styrène.

6. Composition selon l'une quelconque des revendications qui précèdent **caractérisée en ce que** le polymère séquencé est non élastomère.

**7.** Composition selon la revendication précédente **caractérisée en ce que** les première et seconde séquences sont incompatibles l'une avec l'autre.

**8.** Composition selon l'une quelconque des revendications précédentes, **caractérisée par le fait que** le polymère séquencé comprend au moins une première séquence ayant une température de transition vitreuse (Tg) supérieure ou égale à 40°C et au moins une deuxième séquence ayant une température de transition vitreuse inférieure ou égale à 20°C.

**9.** Composition selon la revendication précédente, **caractérisée par le fait que** la proportion de la première séquence va de 20 à 90% en poids du polymère, mieux de 30 à 80% et encore mieux de 50 à 70%.

**10.** Composition selon la revendication 8 ou 9, **caractérisée par le fait que** la proportion de la deuxième séquence ayant une Tg inférieure ou égale à 20°C va de 5 à 75% en poids du polymère, mieux de 15 à 50% et encore mieux de 25 à 45%.

**11.** Composition selon la revendication 1 à 7, **caractérisée par le fait que** le polymère séquencé comprend au moins une première séquence ayant une température de transition vitreuse (Tg) comprise entre 20 et 40°C et au moins une deuxième séquence ayant une température de transition vitreuse inférieure ou égale à 20°C ou une température de transition vitreuse supérieure ou égale à 40°C.

**12.** Composition selon la revendication précédente, **caractérisée par le fait que** la proportion de la première séquence ayant une Tg comprise entre 20 et 40°C va de 10 à 85% en poids du polymère, mieux de 30 à 80% et encore mieux de 50 à 70%.

**13.** Composition selon la revendication 11 ou 12, **caractérisée par le fait que** la deuxième séquence a une Tg supérieure ou égale à 40 °C.

**14.** Composition selon l'une quelconque des revendications 11 à 13, **caractérisée par le fait que** la proportion de la deuxième séquence ayant une Tg supérieure ou égale à 40°C va de 10 à 85%, de préférence de 20 à 70% et mieux de 30 à 70% en poids du polymère.

**15.** Composition selon la revendication 11 ou 12, **caractérisée par le fait que** la deuxième séquence a une Tg inférieure ou égale à 20 °C.

**16.** Composition selon l'une des revendications 1 à 9 et 15, **caractérisé par le fait que** la proportion de la séquence ayant une température de transition vitreuse inférieure ou égale à 20°C va de 20 à 90% en poids du polymère, mieux de 30 à 80% et encore mieux de 50 à 70%.

**17.** Composition selon l'une quelconque des revendications précédentes, **caractérisée par le fait que** la séquence ayant une Tg supérieure ou égale à 40°C est issue en totalité ou en partie de un ou plusieurs monomères dont l'homopolymère a une température de transition vitreuse supérieure ou égale à 40°C, notamment une Tg allant de 40 à 150 °C, de préférence supérieure ou égale à 50°C, notamment allant de 50°C à 120°C, et préférentiellement supérieure ou égale à 60°C, notamment allant de 60°C à 120°C.

**18.** Composition selon la revendication précédente, **caractérisée par le fait que** la séquence ayant une Tg supérieure ou égale à 40 °C est un copolymère issu de monomères dont l'homopolymère a une température de transition vitreuse supérieure ou égale à 40°C.

**19.** Composition selon l'une des revendications 17 ou 18, **caractérisé par le fait que** les monomères dont l'homopolymère a une température de transition vitreuse supérieure ou égale à 40°C sont choisis parmi les monomères suivants :

- les méthacrylates de formule $CH_2 = C(CH_3)\text{-}COOR_1$
dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,
- les acrylates de formule $CH_2 = CH\text{-}COOR_2$

dans laquelle $R_2$ représente un groupe cycloalkyle en $C_4$ à $C_{12}$ tel que l'acrylate d'isobornyle ou un groupe tertio

butyle,

- les (méth)acrylamides de formule :

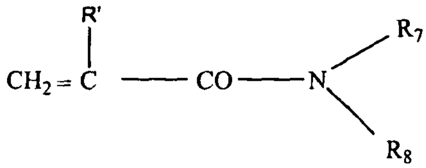

où $R_7$ et $R_8$ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et R' désigne H ou méthyle.

- et leurs mélanges.

**20.** Composition selon l'une quelconque des revendications 16 à 19, **caractérisée par le fait que** les monomères dont l'homopolymère a une température de transition vitreuse supérieure ou égale à 40°C sont choisis parmi le méthacrylate de méthyle, le méthacrylate d'isobutyle, le (méth)acrylate d'isobornyle et leurs mélanges.

**21.** Composition selon l'une quelconque des revendications 1 à 17 et 18 à 20, **caractérisée par le fait que** la séquence ayant une Tg supérieure ou égale à 40°C est un homopolymère.

**22.** Composition selon l'une quelconque des revendications 1 à 11 et 15, 16, **caractérisée par le fait que** la séquence ayant une Tg inférieure ou égale à 20°C est issue en totalité ou en partie de un ou plusieurs monomères dont l'homopolymère a une température de transition vitreuse inférieure ou égale à 20°C, notamment allant de -100 à 20°C, de préférence inférieure ou égale à 15°C, notamment allant de -80°C à 15°C et préférentiellement inférieure ou égale à 10°C, notamment allant de -50°C à 0°C.

**23.** Composition selon la revendication précédente, **caractérisée par le fait que** les monomères dont l'homopolymère a une température de transition vitreuse inférieure ou égale à 20°C sont choisis parmi les monomères suivants :

- les acrylates de formule $CH_2 = CHCOOR_3$,
$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule $CH_2 = C(CH_3)\text{-}COOR_4$,
$R_4$ représentant un groupe alkyle non substitué en $C_6$ à $C_{12}$ linéaire ou ramifié,

dans lequel se trouve(nt) éventuellement intercalé(s) un ou plusieurs hétéroatomes choisis parmi 0, N et S;

- les esters de vinyle de formule $R_5\text{-CO-O-CH} = CH_2$

où $R_5$ représente un groupe alkyle en $C_4$ à $C_{12}$ linéaire ou ramifié ;

- les éthers de vinyle et d'alkyle en $C_4$ à $C_{12}$, tels que l'éther de vinyle et de méthyle et l'éther de vinyle et d'éthyl ;
- les N-alkyl en $C_4$ à $C_{12}$ acrylamides, tels que le N-octylacrylamide,
et leurs mélanges.

**24.** Composition selon la revendication 22 ou 23, **caractérisée par le fait que** les monomères dont l'homopolymère a une température de transition vitreuse inférieure ou égale à 20°C sont choisis parmi les acrylates d'alkyle dont la chaîne alkyle comprend de 1 à 10 atomes de carbone, à l'exception du groupe tertiobutyle

**25.** Composition selon l'une quelconque des revendications 12 à 17 et 20 à 24, **caractérisée par le fait que** la séquence ayant une température de transition vitreuse inférieure ou égale à 20°C est un homopolymère.

**26.** Composition selon l'une quelconque des revendications précédentes, **caractérisée par le fait que** la séquence

ayant une Tg comprise entre 20 et 40°C est issue en totalité ou en partie de un ou de plusieurs monomères dont l'homopolymère a une température de transition vitreuse comprise entre 20 et 40°C.

**27.** Composition selon l'une quelconque des revendications 1 à 7 et 11 à 26, **caractérisée par le fait que** la séquence ayant une Tg comprise entre 20 et 40 °C est un homopolymère d'un monomère choisi parmi le méthacrylate de n-butyle, l'acrylate de cyclodécyle, l'acrylate de néopentyle, l'isodécylacrylamide.

**28.** Composition selon l'une quelconque des revendications 1 à 7 et 11 à 26, **caractérisée par le fait que** la séquence ayant une Tg comprise entre 20 et 40°C est un copolymère issu en totalité ou en partie de :

- monomères dont l'homopolymère a une Tg supérieure ou égale à 40°C, notamment une Tg allant de 40°C à 150°C, de préférence supérieure ou égale à 50°C, notamment allant de 50 à 120°C, et préférentiellement supérieure ou égale à 60°C, notamment allant de 60°C à 120°C,
- et de monomères dont l'homopolymère a une Tg inférieure ou égale à 20°C, notamment allant de -100 à 20°C, de préférence inférieure ou égale à 15°C, notamment allant de -80°C à 15°C, et préférentiellement inférieure ou égale à 10°C, par exemple allant de -50°C à 0°C.

**29.** Composition selon l'une quelconque des revendications 1 à 6 et 11 à 26 et 28, **caractérisée par le fait que** la séquence ayant une Tg comprise entre 20 et 40°C est issue en totalité ou en partie de monomères choisis parmi le méthacrylate de méthyle, le (méth)acrylate d'isobomyle, le méthacrylate de trifluoroéthyle, l'acrylate de butyle, l'acrylate d'éthyl-2 hexyle et leurs mélanges.

**30.** Composition selon l'une quelconque des revendications 1 à 20 et 22 à 24, et 26, 28, 29, **caractérisée par le fait que** la première séquence et/ou la deuxième séquence comprend au moins un monomère additionnel.

**31.** Composition selon la revendication précédente, **caractérisée par le fait que** le monomère additionnel est choisi parmi les monomères hydrophiles, les monomères à insaturation éthylénique comprenant un ou plusieurs atomes de silicium et leurs mélanges.

**32.** Composition selon la revendication 30 ou 31, **caractérisée par le fait que** le monomère additionnel est choisi parmi :

- les monomères à insaturation(s) éthylénique(s) comprenant au moins une fonction acide carboxylique ou sulfonique
- les méthacrylates de formule $CH_2 = C(CH_3)\text{-}COOR_6$

dans laquelle $R_6$ représente un groupe alkyle linéaire ou ramifié, contenant de 1 à 4 atomes de carbone, ledit groupe alkyle étant substitué par un ou plusieurs substituants choisis parmi le groupe hydroxyle et les atomes d'halogènes,

- les méthacrylates de formule $CH_2 = C(CH_3)\text{-}COOR_9$,
$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi le groupe hydroxyle et les atomes d'halogènes ;
- les acrylates de formule $CH_2 = CHCOOR_{10}$,
$R_{10}$ représentant un groupe alkyle en $C_1$ à $C_{12}$ linéaire ou ramifié substitué par un

ou plusieurs substituants choisis parmi le groupe hydroxyle et les atomes d'halogène, ou $R_{10}$ représente un alkyle ($C_1$-$C_{12}$)-O-POE (polyoxyéthylène) avec répétition du motif oxyéthylène de 5 à 30 fois, ou $R_{10}$ représente un groupement polyoxyéthylèné comprenant de 5 à 30 motifs d'oxyde d'éthylène

- les monomères à insaturation(s) éthylénique(s) comprenant au moins une fonction amine tertiaire,
et leurs mélanges.

**33.** Composition selon l'une quelconque des revendications 30 à 32, **caractérisée par le fait que** le ou les monomères additionnels sont choisis parmi l'acide acrylique, l'acide méthacrylique, le méthacrylate de trifluoroéthyle et leurs mélanges.

**34.** Composition selon l'une des revendications 30 à 33, **caractérisée par le fait que** le ou les monomères additionnel (s) représente(nt) de 1 à 30% en poids du poids total des première et/ou deuxième séquences.

**35.** Composition selon l'une des revendications précédentes, **caractérisée par le fait que** chacune des première et deuxième séquence comprend au moins un monomère choisi parmi les esters d'acide (méth)acrylique, et éventuellement au moins un monomère choisi parmi l'acide (méth)acrylique, et leurs mélanges.

**36.** Composition selon l'une des revendications précédentes, **caractérisée par le fait que** chacune des première et deuxième séquence est issue en totalité d'au moins un monomère choisi parmi les esters d'acide (méth)acrylique, et éventuellement d'au moins un monomère choisi parmi l'acide (méth)acrylique, et leurs mélanges

**37.** Composition selon l'une des revendications précédentes, **caractérisée par le fait que** les première et deuxième séquences sont telles que l'écart entre les températures de transition vitreuse (Tg) des première et deuxième séquences est supérieur à 10°C, de préférence supérieur à 20°C, préférentiellement supérieur à 30°C, et plus préférentiellement supérieur à 40°C.

**38.** Composition selon l'une des revendications précédentes, **caractérisée par le fait que** la séquence intermédiaire a une température de transition vitreuse comprise entre les températures de transition vitreuse des première et deuxième séquences.

**39.** Composition selon l'une des revendications précédentes, **caractérisée par le fait que** le polymère séquencé a un indice de polydispersité compris entre 2,8 et 6.

**40.** Composition selon l'une des revendications précédentes, **caractérisée par le fait que** le polymère séquencé a une masse moyenne en poids (Mw) inférieure ou égale à 300 000, de préférence allant de 35 000 à 200 000, et mieux allant de 45 000 à 150 000.

**41.** Composition selon l'une des revendications précédentes, **caractérisée par le fait que** le polymère séquencé a masse moyenne en nombre (Mn) est inférieure ou égale à 70 000, de préférence allant de 10 000 à 60 000, et mieux allant de 12 000 à 50 000.

**42.** Composition selon l'une des revendications précédentes, **caractérisée par le fait que** le polymère séquencé n'est pas soluble à une teneur en matière active d'au moins 1 % en poids dans l'eau ou dans un mélange d'eau et de monoalcools inférieurs linéaires ou ramifiés ayant de 2 à 5 atomes de carbone, sans modification de pH, à température ambiante (25°C).

**43.** Composition selon l'une quelconque des revendications précédentes, **caractérisée par le fait que** le polymère séquencé est présent en une teneur allant de 0,1 % à 90 % en poids, par rapport au poids total de la composition, de préférence allant de 0,5 % à 50 % en poids, et préférentiellement allant de 0,5 % à 30 % en poids.

**44.** Composition selon l'une quelconque des revendications précédentes, **caractérisée par le fait que** le plastifiant est un composé ayant un paramètre de solubilité $\delta_b$ allant de 5,5 à 11 $(J/cm^3)^{1/2}$, de préférence allant de 5,5 à 11, préférentiellement allant de 5,9 à 11, de préférence allant de 7 à 10,5, de préférence allant de 9 à 10, plus préférentiellement allant de 8 à 10 $(J/cm^3)^{1/2}$.

**45.** Composition selon l'une quelconque des revendications précédentes, **caractérisée par le fait que** le plastifiant est un composé ayant un paramètre de solubilité $\delta p$ allant de 1,5 à 4,5 $(J/cm^3)^{1/2}$, de préférence allant de 1,5 à 4 $(J/cm^3)^{1/2}$, de préférence allant de 1,5 à 3,5 $(J/cm^3)^{1/2}$, de préférence encore allant de 2 à 3 $(J/cm^3)^{1/2}$.

**46.** Composition selon l'une quelconque des revendications précédentes, **caractérisée par le fait que** le plastifiant a une masse moléculaire inférieure ou égale à 5000 g/mol, de préférence inférieure ou égale à 2000 g/mol, préférentiellement inférieure ou égale à 1000 g/mol, et plus préférentiellement inférieure ou égale à 900 g/mol. Le plastifiant a avantageusement une masse moléculaire supérieure ou égale à 100 g/mol.

**47.** Composition selon l'une quelconque des revendications précédentes, **caractérisée par le fait que** le plastifiant est un ester.

**48.** Composition selon l'une quelconque des revendications précédentes, **caractérisée par le fait que** le plastifiant est choisi parmi les esters d'au moins un acide carboxylique comprenant 1 à 7 atomes de carbones et d'un polyol comprenant au moins 4 groupes hydroxyles.

**49.** Composition selon la revendication précédente, **caractérisée par le fait que** le polyol est un ose, de préférence un ose cyclisé sous forme d'hémi-acétal.

**50.** Composition selon l'une revendications 48 ou 49, **caractérisée par le fait que** le polyol est choisi parmi le D-ribose, le D-xylose, le L-arabinose, le D-glucose, le D-mannose, le D-galactose, le D-xylulose, le D-fructose.

**51.** Composition selon la revendication 50, **caractérisée par le fait que** le polyol est un mono- ou un polysaccharide comprenant de 1 à 10 oses, de préférence de 1 à 4, de préférence encore un ou deux oses.

**52.** Composition selon la revendication 48 ou 51, **caractérisée par le fait que** le polyol est choisi parmi l'érythritol, le xylitol, le sorbitol, le glucose, le saccharose, le lactose, le maltose.

**53.** Composition selon l'une quelconque des revendications 48, 51, 52, **caractérisée par le fait que** le polyol est le saccharose.

**54.** Composition selon l'une quelconque des revendications 47 à 52, **caractérisée par le fait que** l'ester est un polyol estérifié par au moins deux acides monocarboxyliques différents.

**55.** Composition selon l'une quelconque des revendications 47 à 54, **caractérisée par le fait que** l'acide carboxylique est un acide monocarboxylique comprenant de 1 à 7 atomes de carbones, de préférence de 1 à 5 atomes de carbone.

**56.** Composition selon l'une quelconque des revendications 47 à 55, **caractérisée par le fait que** l'acide carboxylique est choisi parmi les acides acétique, n-propanoïque, isopropanoïque, n-butanoïque, isobutanoïque, tertiobutanoïque, n-pentanoïque et benzoïque.

**57.** Composition selon l'une quelconque des revendications précédentes, **caractérisée par le fait que** le plastifiant est un di-acétate hexa-(2-méthylpropanoate) de saccharose.

**58.** Composition selon l'une quelconque des revendications 1 à 46, **caractérisée par le fait que** le plastifiant est choisi parmi les esters d'acide polycarboxylique aliphatique ou aromatique et d'alcool aliphatique ou aromatique comprenant de 1 à 10 atomes de carbone.

**59.** Composition selon la revendication précédente, **caractérisée par le fait que** :

- l'alcool aliphatique est choisi parmi les alcools R1OH R1 représente méthyle, éthyle, propyle, isopropyle, butyle, hexyle, éthylhexyle, décyle, isodécyle, benzyle,

ou benzyle substitué par un alkyle comprenant 1 à 3 atomes de carbone, et leur mélanges ;

- l'acide polycarboxylique aliphatique ou aromatique comprend de 3 à 12 atomes de carbone, de préférence de 3 à 10 atomes de carbone, de préférence de 3 à 8 atomes de carbone, par exemple 6 ou 8 atomes de carbones.

**60.** Composition selon la revendication 58 ou 59, **caractérisée par le fait que** l'acide polycarboxylique aliphatique ou aromatique est choisi parmi les acides dicarboxyliques et les acides tricarboxyliques.

**61.** Composition selon l'une quelconque des revendications 58 à 60, **caractérisée par le fait que** l'acide polycarboxylique est un diacide carboxylique choisi parmi l' acide succinique, l'acide adipique, l'acide sébacique, et l'acide phtalique.

**62.** Composition selon l'une quelconque des revendications 58 à 60, **caractérisée par le fait que** l'acide polycarboxylique est un acide de formule

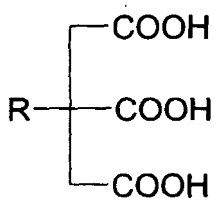

dans laquelle R représente un groupement -H, -OH ou -OCOR' dans lequel R' représente un groupement alkyle ayant de 1 à 6 atomes de carbone, de préférence R représente un groupement $-OCOCH_3$.

**63.** Composition selon l'une quelconque des revendications 58 à 60 et 62, **caractérisée par le fait que** l'acide polycarboxylique est choisi parmi l'acide acétyl-citrique, l'acide butyroyl-citrique, l'acide citrique.

**64.** Composition selon l'une quelconque des revendications 58 à 63, **caractérisée par le fait que** l'ester est choisi parmi l'acétyl-citrate de tributyle, l'acétyl-citrate de triéthyle, l'acétyl-citrate de triéthylhexyle, l'acétyl-citrate de trihexyle, le butyroylcitrate de trihexyle, le citrate de triisodécyle, le citrate de triisopropyle, le citrate de tributyle, le citrate de tri(éthyl-2- hexyle), l'adipate de dibutyle, l'adipate de di(éthyl-2-hexyle), le sébaçate de dibutyle, le sébaçate de di(éthyl-2-hexyle), le sébaçate de diéthyle, le sébaçate de diisopropyle, le succinate de di(éthyl-2-hexyle), le succinate de diéthyle, le phtalate de butyle et de benzyle, le phtalate de dibutyle, le phtalate de diéthylhexyle, le phtalate de diéthyle, le phtalate de diméthyle.

**65.** Composition selon l'une quelconque des revendications 44 à 64, **caractérisée par le fait que** le plastifiant ne comprend de préférence aucun groupe polaire à l'exception du groupe ester, et en particulier ne comprend aucun groupe hydroxyle, les "groupes polaires" étant des groupes polaires ioniques ou non ioniques choisis parmi -COOH ; -OH ; oxyde d'éthylène; oxyde de propylène; - $PO_4$; -NHR ; $-NR_1R_2$ avec $R_1$, $R_2$ représentant un radical alkyle ou alkoxy en $C_1$ à $C_{20-}$ , pouvant être linéaire, ramifié ou cyclique.

**66.** Composition selon l'une quelconque des revendications précédentes, **caractérisée par le fait que** le plastifiant est présent en une teneur allant de 0,1 % à 25% en poids, par rapport au poids total de la composition, de préférence allant de 0,5 % à 15% en poids, et préférentiellement allant de 3 % à 15% en poids.

**67.** Composition selon l'une quelconque des revendications précédentes, **caractérisée par le fait que** le polymère séquencé et le plastifiant sont présents en une teneur telle que le rapport massique entre le polymère séquencé et le plastifiant est compris entre 0,5 et 100, de préférence entre 1 et 50, de préférence entre 1 et 10, de préférence encore entre 1 à 5.

**68.** Composition selon l'une quelconque des revendications précédentes, **caractérisée par le fait que** qu'elle comprend un huile volatile.

**69.** Composition selon l'une quelconque des revendications précédentes, **caractérisée par le fait qu'**elle comprend un huile volatile choisie parmi l'octaméthylcyclotétrasiloxane, le décaméthylcyclopentasiloxane, le dodécaméthylcyclohexasiloxane, l'heptaméthyl-hexyltrisiloxane, l'heptaméthyloctyltrisiloxane, l'octaméthyltrisiloxane, le décaméthyltétrasiloxane, l'isododécane, l'isodécane, l'isohexadécane.

**70.** Composition selon la revendication 68 ou 69, **caractérisée par le fait que** l'huile volatile est présente en une teneur allant de 0,1 % à 90 % en poids, par rapport au poids total de la composition, de préférence allant de 1 % à 70 % en poids, et préférentiellement allant de 5 % à 50 % en poids.

**71.** Composition selon l'une quelconque des revendications précédentes, **caractérisée par le fait qu'**elle comprend une huile non volatile.

**72.** Composition selon la revendication précédente, **caractérisée par le fait que** l'huile non volatile est choisie parmi les huiles non volatiles hydrocarbonées, les huiles non volatiles siliconées.

**73.** Composition selon l'une quelconque des revendications précédentes, **caractérisée par le fait que** l'huile non volatile est présente en une teneur allant de 0,1 % à 20 % en poids, par rapport au poids total de la composition,

de préférence allant de 1% à 15 % en poids, et préférentiellement allant de 1 % à 10 % en poids.

**74.** Composition selon l'une quelconque des revendications précédentes, **caractérisée par le fait qu'**elle comprend au moins un corps gras solides à température ambiante choisi parmi les cires, les corps gras pâteux, les gommes et leurs mélanges.

**75.** Composition selon l'une quelconque des revendications précédentes, **caractérisée par le fait qu'**elle contient de 0,1 à 50 % en poids de cires, par rapport au poids total de la composition, et de préférence de 1 à 30 % en poids.

**76.** Composition selon l'une quelconque des revendications précédentes, **caractérisée par le fait qu'**elle comprend une matière colorante.

**77.** Composition selon l'une quelconque des revendications précédentes, **caractérisée par le fait qu'**elle comprend un ingrédient cosmétique choisi parmi les polymères filmogènes additionnels, les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, ou leurs mélanges.

**78.** Composition cosmétique selon l'une quelconque des revendications précédentes, **caractérisée par le fait qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre.

**79.** Composition cosmétique selon l'une quelconque des revendications précédentes, **caractérisée par le fait qu'**elle se présente sous forme anhydre.

**80.** Composition cosmétique selon l'une quelconque des revendications précédentes, **caractérisée par le fait qu'**il s'agit d'une composition de maquillage ou de soin des matières kératiniques.

**81.** Composition selon l'une quelconque des revendications précédentes, **caractérisée par le fait que** la composition est un produit de maquillage des lèvres.

**82.** Ensemble cosmétique comprenant :

a) un récipient délimitant au moins un compartiment, ledit récipient étant fermé par un élément de fermeture ; et
b) une composition disposée à l'intérieur dudit compartiment, la composition étant conforme à l'une quelconque des revendications qui précèdent.

**83.** Ensemble cosmétique selon la revendication 82, **caractérisé par le fait que** le récipient est formé, au moins pour partie, en au moins un matériau thermoplastique.

**84.** Ensemble cosmétique selon la revendication 82, **caractérisé par le fait que** le récipient est formé, au moins pour partie, en au moins un matériau non thermoplastique, notamment en verre ou en métal.

**85.** Ensemble selon l'une quelconque des revendications 82 à 84, **caractérisé par le fait que**, en position fermée du récipient, l'élément de fermeture est vissé sur le récipient.

**86.** Ensemble selon l'une quelconque des revendications 82 à 84, **caractérisé par le fait que**, en position fermée du récipient, l'élément de fermeture est couplé au récipient autrement que par vissage, notamment par encliquetage, collage, ou soudage.

**87.** Ensemble selon l'une quelconque des revendications 82 à 86, **caractérisé par le fait que** la composition est sensiblement à la pression atmosphérique à l'intérieur du compartiment.

**88.** Ensemble selon l'une quelconque des revendications 82 à 86, **caractérisé par le fait que** la composition est pressurisée à l'intérieur du récipient.

**89.** Procédé cosmétique de maquillage ou de soin des matières kératiniques comprenant l'application sur les matières kératiniques d'une composition cosmétique selon l'une des revendications 1 à 81.

**90.** Utilisation d'une composition selon l'une des revendications 1 à 81, pour obtenir un dépôt, notamment un maquillage, sur les matières kératiniques, en particulier sur la peau ou les lèvres, souple et/ou confortable au cours du temps.

**Claims**

**1.** Cosmetic composition comprising a cosmetically acceptable organic liquid medium, a block polymer and a plasticizer, **characterized in that**:

- the block polymer is a film-forming linear ethylenic polymer comprising first and second blocks connected to one another via an intermediate segment which is a random polymer comprising at least one constituent monomer of the first block and at least one constituent monomer of the second block, the first block of the polymer being chosen from:

  a) a block having a Tg of greater than or equal to 40°C,
  b) a block having a Tg of less than or equal to 20°C,
  c) a block having a Tg of between 20 and 40°C, and

  the second block being chosen from a category a), b) or c) different from the first block; and said block polymer having a polydispersité index I of greater than or equal to 2.8,
- the plasticizer is a compound, the nature and the amount of which are such that the composition is capable of forming a film having a hardness of less than or equal to 35 seconds, the hardness of the film being measured using a Persoz pendulum according to Standard NF-T-30-016.

**2.** Composition according to Claim 1, **characterized in that** the film has a hardness of less than 30 seconds, preferably of less than 25 seconds.

**3.** Composition according to Claim 1, **characterized in that** the film has a hardness ranging from 8 to 35 seconds, preferably ranging from 10 to 30 seconds and preferentially ranging from 12 to 25 seconds.

**4.** Composition according to any one of the preceding claims, **characterized in that** the plasticizer is a compound having a solubility parameter $\delta_h$ ranging from 5.5 to 11 $(J/cm^3)^{1/2}$.

**5.** Composition according to any one of the preceding claims, **characterized in that** the block polymer is devoid of styrene.

**6.** Composition according to any one of the preceding claims, **characterized in that** the block polymer is non-elastomeric.

**7.** Composition according to the preceding claim, **characterized in that** the first and second blocks are incompatible with one another.

**8.** Composition according to any one of the preceding claims, **characterized in that** the block polymer comprises at least one first block having a glass transition temperature (Tg) of greater than or equal to 40°C and at least one second block having a glass transition temperature of less than or equal to 20°C.

**9.** Composition according to the preceding claim, **characterized in that** the proportion of the first block ranges from 20 to 90% by weight of the polymer, better still from 30 to 80% and even better still from 50 to 70%.

**10.** Composition according to Claim 8 or 9, **characterized in that** the proportion of the second block having a Tg of less than or equal to 20°C ranges from 5 to 75% by weight of the polymer, better still from 15 to 50% and even better still from 25 to 45%.

**11.** Composition according to Claims 1 to 7, **characterized in that** the block polymer comprises at least one first block having a glass transition temperature (Tg) of between 20 and 40°C and at least one second block having a glass

transition temperature of less than or equal to 20°C or a glass transition temperature of greater than or equal to 40°C.

**12.** Composition according to the preceding claim, **characterized in that** the proportion of the first block having a Tg of between 20 and 40°C ranges from 10 to 85% by weight of the polymer, better still from 30 to 80% and even better still from 50 to 70%.

**13.** Composition according to Claim 11 or 12, **characterized in that** the second block has a Tg of greater than or equal to 40°C.

**14.** Composition according to any one of Claims 11 to 13, **characterized in that** the proportion of the second block having a Tg of greater than or equal to 40°C ranges from 10 to 85%, preferably from 20 to 70% and better still from 30 to 70% by weight of the polymer.

**15.** Composition according to Claim 11 or 12, **characterized in that** the second block has a Tg of less than or equal to 20°C.

**16.** Composition according to one of Claims 1 to 9 and 15, **characterized in that** the proportion of the block having a glass transition temperature of less than or equal to 20°C ranges from 20 to 90% by weight of the polymer, better still from 30 to 80% and even better still from 50 to 70%.

**17.** Composition according to any one of the preceding claims, **characterized in that** the block having a Tg of greater than or equal to 40°C results, in all or in part, from one or more monomers, the homopolymer of which has a glass transition temperature of greater than or equal to 40°C, in particular a Tg ranging from 40 to 150°C, preferably of greater than or equal to 50°C, in particular ranging from 50°C to 120°C, and preferentially of greater than or equal to 60°C, in particular ranging from 60°C to 120°C.

**18.** Composition according to the preceding claim, **characterized in that** the block having a Tg of greater than or equal to 40°C is a copolymer resulting from monomers, the homopolymer of which has a glass transition temperature of greater than or equal to 40°C.

**19.** Composition according to either of Claims 17 or 18, **characterized in that** the monomers, the homopolymer of which has a glass transition temperature of greater than or equal to 40°C, are chosen from the following monomers:

- methacrylates of formula $CH_2=C(CH_3)-COOR_1$,

in which $R_1$ represents an unsubstituted linear or branched alkyl group comprising from 1 to 4 carbon atoms, such as a methyl, ethyl, propyl or isobutyl group, or $R_1$ represents a $C_4$ to $C_{12}$ cycloalkyl group,

- acrylates of formula $CH_2=CH-COOR_2$,

in which $R_2$ represents a $C_4$ to $C_{12}$ cycloalkyl group, such as isobornyl acrylate, or a tert-butyl group,

- (meth)acrylamides of formula:

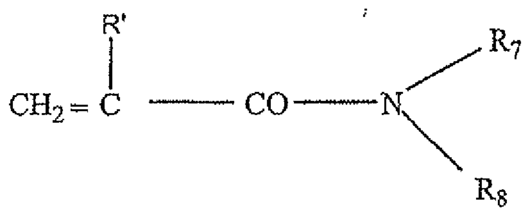

where $R_7$ and $R_8$, which are identical or different, each represent a hydrogen atom or a linear or branched alkyl group of 1 to 12 carbon atoms, such as an n-butyl, t-butyl, isopropyl, isohexyl, isooctyl or isononyl group, or $R_7$ represents H and $R_8$ represents a 1,1-dimethyl-3-oxobutyl group, and R' denotes H or methyl,

- and their mixtures.

**20.** Composition according to any one of Claims 16 to 19, **characterized in that** the monomers, the homopolymer of which has a glass transition temperature of greater than or equal to 40°C, are chosen from methyl methacrylate, isobutyl methacrylate, isobornyl (meth)acrylate and their mixtures.

**21.** Composition according to any one of Claims 1 to 17 and 18 to 20, **characterized in that** the block having a Tg of greater than or equal to 40°C is a homopolymer.

**22.** Composition according to any one of Claims 1 to 11 and 15, 16, **characterized in that** the block having a Tg of less than or equal to 20°C results, in all or in part, from one or more monomers, the homopolymer of which has a glass transition temperature of less than or equal to 20°C, in particular ranging from -100 to 20°C, preferably of less than or equal to 15°C), in particular ranging from -80°C to 15°C and preferentially of less than or equal to 10°C, in particular ranging from -50°C to 0°C.

**23.** Composition according to the preceding claim, **characterized in that** the monomers, the homopolymer of which has a glass transition temperature of less than or equal to 20°C, are chosen from the following monomers:

- acrylates of formula $CH_2$=$CHCOOR_3$,
$R_3$ representing an unsubstituted linear or branched $C_1$ to $C_{12}$ alkyl group, with the exception of the tert-butyl group, in which one or more heteroatoms chosen from O, N or S is(are) optionally inserted,
- methacrylates of formula $CH_2$=$C(CH_3)$-$COOR_4$,
$R_4$ representing an unsubstituted linear or branched $C_6$ to $C_{12}$ alkyl group in which one or more heteroatoms chosen from O, N and S is (are) optionally inserted,
- vinyl esters of formula $R_5$-CO-O-CH=$CH_2$,

where $R_5$ represents a linear or branched $C_4$ to $C_{12}$ alkyl group,

- $C_4$ to $C_{12}$ alkyl vinyl ethers, such as methyl vinyl ether and ethyl vinyl ether,
- N-($C_4$ to $C_{12}$ alkyl) acrylamides, such as N-octylacrylamide,
- and their mixtures.

**24.** Composition according to Claim 22 or 23, **characterized in that** the monomers, the homopolymer of which has a glass transition temperature of less than or equal to 20°C, are chosen from alkyl acrylates, the alkyl chain of which comprises from 1 to 10 carbon atoms, with the exception of the tert-butyl group.

**25.** Composition according to any one of Claims 12 to 17 and 20 to 24, **characterized in that** the block having a glass transition temperature of less than or equal to 20°C is a homopolymer.

**26.** Composition according to any one of the preceding claims, **characterized in that** the block having a Tg of between 20 and 40°C results, in all or in part, from one or more monomers, the homopolymer of which has a glass transition temperature of between 20 and 40°C.

**27.** Composition according to any one of Claims 1 to 7 and 11 to 26, **characterized in that** the block having a Tg of between 20 and 40°C is a homopolymer of a monomer chosen from n-butyl methacrylate, cyclodecyl acrylate, neopentyl acrylate or isodecylacrylamide.

**28.** Composition according to any one of Claims 1 to 7 and 11 to 26, **characterized in that** the block having a Tg of between 20 and 40°C is a copolymer resulting, in all or in part, from:

- monomers, the homopolymer of which has a Tg of greater than or equal to 40°C, in particular a Tg ranging from 40°C to 150°C, preferably of greater than or equal to 50°C, in particular ranging from 50 to 120°C, and preferentially of greater than or equal to 60°C, in particular ranging from 60°C to 120°C,
- and monomers, the homopolymer of which has a Tg of less than or equal, to 20°C, in particular ranging from - 100 to 20°C, preferably of less than or equal to 15°C, in particular ranging from -80°C to 15°C, and preferentially of less than or equal to 10°C, for example ranging from -50°C to 0°C.

**29.** Composition according to any one of Claims 1 to 6 and 11 to 26 and 28, **characterized in that** the block having a

Tg of between 20 and 40°C results, in all or in part, from monomers chosen from methyl methacrylate, isobornyl (meth)acrylate, trifluoroethyl methacrylate, butyl acrylate, 2-ethylhexyl acrylate and their mixtures.

**30.** Composition according to any one of Claims 1 to 20 and 22 to 24 and 26, 28 and 29, **characterized in that** the first block and/or the second block comprises at least one additional monomer.

**31.** Composition according to the preceding claim, **characterized in that** the additional monomer is chosen from hydrophilic monomers, monomers with ethylenic unsaturation comprising one or more silicone atoms and their mixtures.

**32.** Composition according to Claim 30 or 31, **characterized in that** the additional monomer is chosen from:

- monomers with ethylenic unsaturation(s) comprising at least one carboxylic or sulphonic acid functional group,
- methacrylates of formula $CH_2=C(CH_3)-COOR_6$,

in which $R_6$ represents a linear or branched alkyl group comprising from 1 to 4 carbon atoms, said alkyl group being substituted by one or more substituents chosen from the hydroxyl group and halogen atoms,

- methacrylates of formula $CH_2=C(CH_3)-COOR_9$,
$R_9$ representing a linear or branched $C_6$ to $C_{12}$ alkyl group into which one or more heteroatoms chosen from O, N and S is (are) optionally inserted, said alkyl group being substituted by one or more substituents chosen from the hydroxyl group and halogen atoms,
- acrylates of formula $CH_2=CHCOOR_{10}$,
$R_{10}$ representing a linear or branched $C_1$ to $C_{12}$ alkyl group substituted by one or more substituents chosen from the hydroxyl group and halogen atoms, or $R_{10}$ representing a $(C_1-C_{12})$alkyl-O-POE (polyoxyethylene) with repetition of the oxyethylene unit from 5 to 30 times, or $R_{10}$ representing a polyoxyethylene group comprising from 5 to 30 ethylene oxide units,
- monomers with ethylenic unsaturation(s) comprising at least one tertiary gamine functional group,
- and their mixtures.

**33.** Composition according to any one of Claims 30 to 32, **characterized in that** the additional monomer or monomers are chosen from acrylic acid, methacrylic acid, trifluoroethyl methacrylate and their mixtures.

**34.** Composition according to one of Claims 30 to 33, **characterized in that** the additional monomer or monomers represent(s) from 1 to 30% by weight of the total weight of the first and/or second blocks.

**35.** Composition according to one of the preceding claims, **characterized in that** each of the first and second blocks comprises at least one monomer chosen from (meth)acrylic acid esters and optionally at least one monomer chosen from (meth)acrylic acid, and their mixtures.

**36.** Composition according to one of the preceding claims, **characterized in that** each of the first and second blocks results, in all, from at least one monomer chosen from (meth)acrylic acid esters and optionally from at least one monomer chosen from (meth)acrylic acid, and their mixtures.

**37.** Composition according to one of the preceding claims, **characterized in that** the first and second blocks are such that the difference between the glass transition temperatures (Tg) of the first and second blocks is greater than 10°C, preferably greater than 20°C, preferentially greater than 30°C and more preferentially greater than 40°C.

**38.** Composition according to one of the preceding claims, **characterized in that** the intermediate block has a glass transition temperature between the glass transition temperatures of the first and second blocks.

**39.** Composition according to one of the preceding claims, **characterized in that** the block polymer has a polydispersity index of between 2.8 and 6.

**40.** Composition according to one of the preceding claims, **characterized in that** the block polymer has a weight-average mass (Mw) of less than or equal to 300 000, preferably ranging from 35 000 to 200 000 and better still ranging from 45 000 to 150 000.

**41.** Composition according to one of the preceding claims, **characterized in that** the block polymer has a number-average mass (Mn) of less than or equal to 70 000, preferably ranging from 10 000 to 60 000 and better still ranging from 12 000 to 50 000.

**42.** Composition according to one of the preceding claims, **characterized in that** the block polymer is insoluble, at an active material content of at least 1% by weight, in water or in a mixture of water and of linear or branched lower monoalcohols having from 2 to 5 carbon atoms, without modification of pH, at ambient temperature (25°C).

**43.** Composition according to any one of the preceding claims, **characterized in that** the block polymer is present in a content ranging from 0.1% to 90% by weight, with respect to the total weight of the composition, preferably ranging from 0.5% to 50% by weight and preferentially ranging from 0.5% to 30% by weight.

**44.** Composition according to any one of the preceding claims, **characterized in that** the plasticizer is a compound having a solubility parameter $\delta_h$ ranging from 5. 5 to 11 $(J/cm^3)^{1/2}$, preferably ranging from 5.5 to 11, preferentially ranging from 5.9 to 11, preferably ranging from 7 to 10.5, preferably ranging from 9 to 10, more preferentially ranging from 8 to 10 $(J/cm^3)^{1/2}$.

**45.** Composition according to any one of the preceding claims, **characterized in that** the plasticizer is a compound having a solubility parameter $\delta_p$ ranging from 1.5 to 4.5 $(J/cm^3)^{1/2}$, preferably ranging from 1.5 to 4 $(J/cm^3)^{1/2}$, preferably ranging from 1.5 to 3.5 $(J/cm^3)^{1/2}$, preferably again ranging from 2 to 3 $(J/cm^3)^{1/2}$.

**46.** Composition according to any one of the preceding claims, **characterized in that** the plasticizer has a molecular mass of less than or equal to 5000 g/mol, preferably of less than or equal to 2000 g/mol, preferentially of less than or equal to 1000 g/mol and more preferentially of less than or equal to 900 g/mol, the plasticizer advantageously having a molecular mass of greater than or equal to 100 g/mol.

**47.** Composition according to any one of the preceding claims, **characterized in that** the plasticizer is an ester.

**48.** Composition according to any one of the preceding claims, **characterized in that** the plasticizer is chosen from esters of at least one carboxylic acid comprising 1 to 7 carbon atoms and of a polyol comprising at least 4 hydroxyl groups.

**49.** Composition according to the preceding claim, **characterized in that** the polyol is a monosaccharide, preferably a cyclized monosaccharide in the hemiacetal form.

**50.** Composition according to either of Claims 48 and 49, **characterized in that** the polyol is chosen from D-ribose, D-xylose, L-arabinose, D-glucose, D-mannose, D-galactose, D-xylulose or D-fructose.

**51.** Composition according to Claim 50, **characterized in that** the polyol is a mono- or polysaccharide comprising from 1 to 10 monosaccharide units, preferably from 1 to 4, preferably again one or two monosaccharide units.

**52.** Composition according to Claim 48 or 51, **characterized in that** the polyol is chosen from erythritol, xylitol, sorbitol, glucose, sucrose, lactose or maltose.

**53.** Composition according to any one of Claims 48, 51 and 52, **characterized in that** the polyol is sucrose.

**54.** Composition according to any one of Claims 47 to 52, **characterized in that** the ester is a polyol esterified by at least two different monocarboxylic acids.

**55.** Composition according to any one of Claims 47 to 54, **characterized in that** the carboxylic acid is a monocarboxylic acid comprising from 1 to 7 carbon atoms, preferably from 1 to 5 carbon atoms.

**56.** Composition according to any one of Claims 47 to 55, **characterized in that** the carboxylic acid is chosen from acetic, n-propanoic, isopropanoic, n-butanoic, isobutanoic, tert-butanoic, n-pentanoic and benzoic acids.

**57.** Composition according to any one of the preceding claims, **characterized in that** the plasticizer is a sucrose diacetate hexa(2-methylpropanoate).

**58.** Composition according to any one of Claims 1 to 46, **characterized in that** the plasticizer is chosen from esters of

an aliphatic or aromatic polycarboxylic acid and of an aliphatic or aromatic alcohol comprising from 1 to 10 carbon atoms.

**59.** Composition according to the preceding claim, **characterized in that**:

- the aliphatic alcohol is chosen from alcohols $R_1OH$, $R_1$ representing methyl, ethyl, propyl, isopropyl, butyl, hexyl, ethylhexyl, decyl, isodecyl, benzyl or benzyl substituted by an alkyl comprising 1 to 3 carbon atoms, and their mixtures;
- the aliphatic or aromatic polycarboxylic acid comprises from 3 to 12 carbon atoms, preferably from 3 to 10 carbon atoms, preferably from 3 to 8 carbon atoms, for example 6 or 8 carbon atoms.

**60.** Composition according to Claim 58 or 59, **characterized in that** the aliphatic or aromatic polycarboxylic acid is chosen from dicarboxylic acids and tricarboxylic acids.

**61.** Composition according to any one of Claims 58 to 60, **characterized in that** the polycarboxylic acid is a dicarboxylic acid chosen from succinic acid, adipic acid, sebacic acid and phthalic acid.

**62.** Composition according to any one of Claims 58 to 60, **characterized in that** the polycarboxylic acid is an acid of formula:

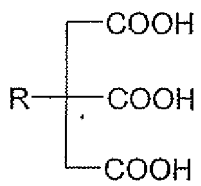

in which R represents an -H, -OH or -OCOR' group in which R' represents an alkyl group having from 1 to 6 carbon atoms, R preferably representing an $-OCOCH_3$ group.

**63.** Composition according to any one of Claims 58 to 60 and 62, **characterized in that** the polycarboxylic acid is chosen from acetylcitric acid, butyroylcitric acid or citric acid.

**64.** Composition according to any one of Claims 58 to 63, **characterized in that** the ester is chosen from tributyl acetylcitrate, triethyl acetylcitrate, triethylhexyl acetylcitrate, trihexyl acetylcitrate, trihexyl butyroylcitrate, triisodecyl citrate, triisopropyl citrate, tributyl citrate, tri(2-ethylhexyl) citrate, dibutyl adipate, di(2-ethylhexyl) adipate, dibutyl sebacate, di(2-ethylhexyl) sebacate, diethyl sebacate, diisopropyl sebacate, di(2-ethylhexyl) succinate, diethyl succinate, butyl benzyl phthalate, dibutyl phthalate, diethylhexyl phthalate, diethyl phthalate or dimethyl phthalate.

**65.** Composition according to any one of Claims 44 to 64, **characterized in that** the plasticizer preferably does not comprise any polar group with the exception of the ester group and in particular does not comprise any hydroxyl group, the "polar groups" being ionic or nonionic polar groups chosen from -COOH, -OH, ethylene oxide, propylene oxide, $-PO_4$, -NHR or $-NR_1R_2$ with $R_1$ and $R_2$ representing a $C_1$ to $C_{20}$ alkyl or alkoxy radical which can be linear, branched or cyclic.

**66.** Composition according to any one of the preceding claims, **characterized in that** the plasticizer is present in a content ranging from 0.1% to 25% by weight, with respect to the total weight of the composition, preferably ranging from 0.5% to 15% by weight and preferentially ranging from 3% to 15% by weight.

**67.** Composition according to any one of the preceding claims, **characterized in that** the block polymer and the plasticizer are present in a content such that the ratio by weight of the block polymer to the plasticizer is between 0.5 and 100, preferably between 1 and 50, preferably between 1 and 10, preferably again between 1 and 5.

**68.** Composition according to any one of the preceding claims, **characterized in that** it comprises a volatile oil.

**69.** Composition according to any one of the preceding claims, **characterized in that** it comprises a volatile oil chosen from octamethylcyclotetrasiloxane, decamethylcyclopentasiloxane, dodecamethylcyclohexasiloxane, heptamethylhexyltrisiloxane, heptamethyloctyltrisiloxane, octamethyltrisiloxane, decamethyltetrasiloxane, isododecane, isodecane or isohexadecane.

**70.** Composition according to Claim 68 or 69, **characterized in that** the volatile oil is present in a content ranging from 0.1% to 90% by weight, with respect to the total weight of the composition, preferably ranging from 1% to 70% by weight and preferentially ranging from 5% to 50% by weight.

**71.** Composition according to any one of the preceding claims, **characterized in that** it comprises a nonvolatile oil.

**72.** Composition according to the preceding claim, **characterized in that** the nonvolatile oil is chosen from nonvolatile hydrocarbon oils or nonvolatile silicone oils.

**73.** Composition according to any one of the preceding claims, **characterized in that** the nonvolatile oil is present in a content ranging from 0.1% to 20% by weight, with respect to the total weight of the composition, preferably ranging from 1% to 15% by weight and preferentially ranging from 1% to 10% by weight.

**74.** Composition according to any one of the preceding claims, **characterized in that** it comprises at least one fatty substance which is solid at ambient temperature chosen from waxes, pasty fatty substances, gums and their mixtures.

**75.** Composition according to any one of the preceding claims, **characterized in that** it comprises from 0.1 to 50% by weight of waxes, with respect to the total weight of the composition, and preferably from 1 to 30% by weight.

**76.** Composition according to any one of the preceding claims, **characterized in that** it comprises a coloring material.

**77.** Composition according to any one of the preceding claims, **characterized in that** it comprises a cosmetic ingredient chosen from additional film-forming polymers, vitamins, thickeners, trace elements, softeners, sequestering agents, fragrances, basifying or acidifying agents, preservatives, sunscreen agents, surfactants, antioxidants, agents for combatting hair loss, antidandruff agents, propellants or their mixtures.

**78.** Cosmetic composition according to any one of the preceding claims, **characterized in that** it is provided in the form of a suspension, dispersion, solution, gel, emulsion, in particular oil-in-water (O/W) or water-in-oil (W/O) or multiple (W/O/W or polyol/O/W or O/w/O) emulsion, cream, foam, dispersion of vesicles, in particular of ionic or nonionic lipids, two-phase or multiphase lotion, spray, powder or paste, in particular soft paste or anhydrous paste.

**79.** Cosmetic composition according to any one of the preceding claims, **characterized in that** it is provided in the anhydrous form.

**80.** Cosmetic composition according to any one of the preceding claims, **characterized in that** it is a composition for making up or caring for keratinous substances.

**81.** Composition according to any one of the preceding claims, **characterized in that** the composition is a product for making up the lips.

**82.** Cosmetic combination comprising:

a) a container delimiting at least one compartment, said container being closed by a closing element; and
b) a composition positioned inside said compartment, the composition being in accordance with any one of the preceding claims.

**83.** Cosmetic combination according to Claim 82, **characterized in that** the container is formed, at least partially, of at least one thermoplastic material.

**84.** Cosmetic combination according to Claim 82, **characterized in that** the container is formed, at least partially, of at least one nonthermoplastic material, in particular of glass or of metal.

**85.** Combination according to any one of Claims 82 to 84, **characterized in that**, in the closed position of the container, the closing element is screwed to the container.

**86.** Combination according to any one of Claims 82 to 84, **characterized in that**, in the closed position of the container, the closing element is coupled to the container other than by screwing, in particular by snapping, adhesive bonding or welding.

**87.** Combination according to any one of Claims 82 to 86, **characterized in that** the composition is substantially at atmospheric pressure inside the compartment.

**88.** Combination according to any one of Claims 82 to 86, **characterized in that** the composition is pressurized inside the container.

**89.** Cosmetic process for making up or caring for keratinous substances, comprising the application to the keratinous substances of a cosmetic composition according to one of Claims 1 to 81.

**90.** Use of a composition according to one of Claims 1 to 81, for producing a deposited layer, in particular a makeup, on keratinous substances, in particular on the skin or lips, which is flexible and/or comfortable over time.

## Patentansprüche

**1.** Kosmetische Zusammensetzung, die ein kosmetisch akzeptables, flüssiges organisches Medium, ein Sequenzpolymer und einen Weichmacher enthält, **dadurch gekennzeichnet, dass**:

- das Sequenzpolymer ein filmbildendes, lineares, ethylenisches Polymer ist, das eine erste Sequenz und eine zweite Sequenz enthält, die über ein Zwischensegment aneinander gebunden sind, bei dem es sich um ein statistisches Polymer handelt, das mindestens ein die erste Sequenz aufbauendes Monomer und mindestens ein die zweite Sequenz aufbauendes Monomer umfasst,

wobei die erste Sequenz des Polymers ausgewählt ist unter:

a) einer Sequenz mit einer Tg von größer oder gleich 40 °C;
b) einer Sequenz mit einer Tg von kleiner oder gleich 20 °C;
c) einer Sequenz mit einer Tg zwischen 20 und 40 °C;

und die zweite Sequenz aus einer von der ersten Sequenz verschiedenen Gruppe a), b) oder c) ausgewählt ist; wobei das Sequenzpolymer einen Polydispersitätsindex I größer oder gleich 2,8 aufweist,

- wobei der Weichmacher eine Verbindung ist, deren Art und Menge so sind, dass die Zusammensetzung befähigt ist, einen Film mit einer Härte von kleiner oder gleich 35 Sekunden zu bilden, wobei die Härte des Films mit Hilfe eines Persoz-Pendels gemäß der Norm NF-T-30-016 ermittelt wird.

**2.** Zusammensetzung nach Anspruch 1, **dadurch gekennzeichnet, dass** der Film eine Härte von kleiner oder gleich 30 Sekunden und vorzugsweise von kleiner oder gleich 25 Sekunden aufweist.

**3.** Zusammensetzung nach Anspruch 1, **dadurch gekennzeichnet, dass** der Film eine Härte im Bereich von 8 bis 35 Sekunden, vorzugsweise im Bereich von 10 bis 30 Sekunden und noch bevorzugter im Bereich von 12 bis 25 Sekunden aufweist.

**4.** Zusammensetzung nach einem der vorhergehenden Ansprüche, **dadurch gekennzeichnet, dass** der Weichmacher eine Verbindung mit einem Solubilitätsparameter $\delta_h$ im Bereich von 5,5 bis 11 $(J/cm^3)^{1/2}$ ist.

**5.** Zusammensetzung nach einem der vorhergehenden Ansprüche, **dadurch gekennzeichnet, dass** das Sequenzpolymer kein Styrol aufweist.

**6.** Zusammensetzung nach einem der vorhergehenden Ansprüche, **dadurch gekennzeichnet, dass** das Sequenzpolymer nicht elastomer ist.

**7.** Zusammensetzung nach dem vorhergehenden Anspruch, **dadurch gekennzeichnet, dass** die erste und zweite Sequenz nicht miteinander kompatibel sind.

**8.** Zusammensetzung nach einem der vorhergehenden Ansprüche, **dadurch gekennzeichnet, dass** das Sequenzpolymer zumindest eine erste Sequenz mit einer Glasübergangstemperatur (Tg) von größer oder gleich 40 °C und zumindest eine zweite Sequenz mit einer Glasübergangstemperatur von kleiner oder gleich 20 °C umfasst.

**9.** Zusammensetzung nach dem vorhergehenden Anspruch, **dadurch gekennzeichnet, dass** der Anteil der ersten Sequenz im Bereich von 20 bis 90 Gew.-% des Polymers, besser im Bereich von 30 bis 80 Gew.-% und besser im Bereich von 50 bis 70 Gew.-% liegt.

**10.** Zusammensetzung nach Anspruch 8 oder 9, **dadurch gekennzeichnet, dass** der Anteil der zweiten Sequenz mit einer Tg von kleiner oder gleich 20 °C im Bereich von 5 bis 75 Gew.-% des Polymers, besser 15 bis 50 Gew.-% und besser 25 bis 45 Gew.-% liegt.

**11.** Zusammensetzung nach Anspruch 1 bis 7, **dadurch gekennzeichnet, dass** das Sequenzpolymer mindestens eine erste Sequenz mit einer Glasübergangstemperatur (Tg) zwischen 20 °C und 40 °C und zumindest eine zweite Sequenz mit einer Glasübergangstemperatur von kleiner oder gleich 20 °C oder einer Glasübergangstemperatur von größer oder gleich 40 °C umfasst.

**12.** Zusammensetzung nach dem vorhergehenden Anspruch, **dadurch gekennzeichnet, dass** der Anteil der ersten Sequenz mit einer Tg zwischen 20 und 40 °C im Bereich von 10 bis 85 Gew.-% des Polymers, besser im Bereich von 30 bis 80 Gew.-% und noch besser im Bereich von 50 bis 70 Gew.-% liegt,

**13.** Zusammensetzung nach Anspruch 11 oder 12, **dadurch gekennzeichnet, dass** die zweite Sequenz eine Tg von größer oder gleich 40 °C aufweist.

**14.** Zusammensetzung nach einem der Ansprüche 11 bis 13, **dadurch gekennzeichnet, dass** der Anteil der zweiten Sequenz mit einer Tg von größer oder gleich 40 °C im Bereich von 10 bis 85 Gew,-%, vorzugsweise im Bereich von 20 bis 70 Gew.-% und besser im Bereich von 30 bis 70 Gew.-% des Polymers liegt.

**15.** Zusammensetzung nach Anspruch 11 oder 12, **dadurch gekennzeichnet, dass** die zweite Sequenz eine Tg von kleiner oder gleich 20 °C aufweist.

**16.** Zusammensetzung nach einem der Ansprüche 1 bis 9 und 15, **dadurch gekennzeichnet, dass** der Anteil der Sequenz mit einer Glasübergangstemperatur von kleiner oder gleich 20 °C im Bereich von 20 bis 90 Gew.-% des Polymers, besser im Bereich von 30 bis 80 Gew.-% und noch besser im Bereich von 50 bis 70 Gew.-% liegt.

**17.** Zusammensetzung nach einem der vorhergehenden Ansprüche, **dadurch gekennzeichnet, dass** die Sequenz, die eine Tg von 40 °C oder darüber aufweist, ganz oder teilweise von einem oder mehreren Monomeren abgeleitet ist, deren Homopolymer eine Glasübergangstemperatur von größer oder gleich 40 °C, insbesondere im Bereich von 40 bis 150 °C, vorzugsweise von 50 °C oder darüber, insbesondere im Bereich von 50 bis 120 °C und bevorzugt von 60 °C oder darüber, insbesondere im Bereich von 60 bis 120 °C aufweist.

**18.** Zusammensetzung nach dem vorhergehenden Anspruch, **dadurch gekennzeichnet, dass** die Sequenz, die eine Tg von 40 °C oder darüber aufweist, ein Copolymer ist, das von Monomeren abgeleitet ist, deren Homopolymer eine Glasübergangstemperatur von 40 °C oder darüber aufweist.

**19.** Zusammensetzung nach einem der Ansprüche 17 oder 18, **dadurch gekennzeichnet, dass** die Monomere, deren Homopolymer eine Glasübergangstemperatur von größer oder gleich 40 °C aufweist, unter den folgenden Monomeren ausgewählt sind:

- Methacrylaten der Formel $CH_2=C(CH_3)-COOR_1$,

worin $R_1$ eine lineare oder verzweigte, unsubstituierte Alkylgruppe mit 1 bis 4 Kohlenstoffatomen, wie Methyl, Ethyl, Propyl oder Isobutyl, bedeutet oder $R_1$ eine Cycloalkylgruppe mit 4 bis 12 Kohlenstoffatomen ist,

- Acrylaten der Formel $CH_2=CH-COOR_2$,

worin $R_2$ eine Cycloalkylgruppe mit 4 bis 12 Kohlenstoffatomen, wie Isobornylacrylat, oder tert-Butyl bedeutet,

- (Meth)acrylamiden der Formel:

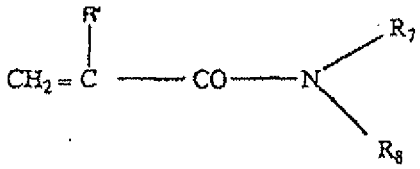

worin $R_7$ und $R_8$, die gleich oder verschieden sind, jeweils ein Wasserstoffatom oder eine lineare oder verzweigte Alkylgruppe mit 1 bis 12 Kohlenstoffatomen, wie n-Butyl, tert-Butyl, Isopropyl, Isohexyl, Isooctyl oder Isononyl, bedeuten.; oder $R_7$ H bedeutet und $R_8$ eine 1,1-Dimethyl-3-oxobutylgruppe ist, und R' H oder Methyl bedeutet;

- und deren Gemischen.

**20.** Zusammensetzung nach einem der Ansprüche 16 bis 19, **dadurch gekennzeichnet, dass** die Monomere, deren Homopolymer eine Glasübergangstemperatur von größer oder gleich 40 °C aufweist, unter Methylmethacrylat, Isobutylmethacrylat und Isobornyl(meth)acrylat und deren Gemischen ausgewählt sind.

**21.** Zusammensetzung nach einem der Ansprüche 1 bis 17 und 18 bis 20, **dadurch gekennzeichnet, dass** die Sequenz, die eine Tg von 40 °C oder darüber aufweist, ein Homopolymer ist.

**22.** Zusammensetzung nach einem der Ansprüche 1 bis 11 und 15, 16, **dadurch gekennzeichnet, dass** die Sequenz, die eine Tg von 20 °C oder darunter aufweist, ganz oder teilweise von einem oder mehreren Monomeren abgeleitet ist, deren Homopolymer eine Glasübergangstemperatur von 20 °C oder darunter, insbesondere im Bereich von --100 bis 20 °C, vorzugsweise von 15 °C oder darunter, insbesondere im Bereich von -80 bis 15 °C und bevorzugt von 10 °C oder darunter, insbesondere im Bereich von -50 bis 0 °C aufweist.

**23.** Zusammensetzung nach dem vorhergehenden Anspruch, **dadurch gekennzeichnet, dass** die Monomere, deren Homopolymer eine Glasübergangstemperatur von kleiner oder gleich 20 °C aufweist, unter den folgenden Monomeren ausgewählt sind:

- Acrylaten der Formel $CH_2=CHCOOR_3$,

worin $R_3$ mit Ausnahme der tert-Butylgruppe eine lineare oder verzweigte, unsubstituierte Alkylgruppe mit 1 bis 12 Kohlenstoffatomen bedeutet, in die optional ein oder mehrere Heteroatome, die unter O, N und S ausgewählt sind, eingebaut ist (sind);

- Methacrylaten der Formel $CH_2=C(CH_3)-COOR_4$,

worin $R_4$ eine lineare oder verzweigte, unsubstituierte Alkylgruppe mit 6 bis 12 Kohlenstoffatomen bedeutet, in die optional ein oder mehrere Heteroatome, die unter O, N und S ausgewählt sind, eingebaut ist (sind);

- Vinylestern der Formel $R_5-CO-O-CH=CH_2$,

worin $R_5$ eine lineare oder verzweigte Alkylgruppe mit 4 bis 12 Kohlenstoffatomen bedeutet;

- Alkyl($C_{4-12}$)vinylethern, wie Methylvinylether und Ethylvinylether;
- N-Alkyl($C_{4-12}$)acrylamiden, wie N-Octylacrylamid,
- und deren Gemischen.

**24.** Zusammensetzung nach Anspruch 22 oder 23, **dadurch gekennzeichnet, dass** die Monomere, deren Homopolymer eine Glasübergangstemperatur von 20 °C oder darunter aufweist, unter den Alkylacrylaten ausgewählt ist, deren Alkylkette 1 bis 10 Kohlenstoffatome aufweist, wobei die Gruppe tert-Butyl ausgenommen ist.

**25.** Zusammensetzung nach einem der Ansprüche 12 bis 17 und 20 bis 24, **dadurch gekennzeichnet, dass** die Sequenz mit einer Glasübergangstemperatur von 20 °C oder darunter ein Homopolymer ist.

**26.** Zusammensetzung nach einem der vorhergehenden Ansprüche, **dadurch gekennzeichnet, dass** die Sequenz mit einer Tg zwischen 20 und 40 °C ganz oder teilweise von einem oder mehreren Monomeren stammt, deren Homopolymer eine Glasübergangstemperatur zwischen 20 und 40 °C aufweist.

**27.** Zusammensetzung nach einem der Ansprüche 1 bis 7 und 11 bis 26, **dadurch gekennzeichnet, dass** die Sequenz mit einer Tg zwischen 20 und 40°C ein Homopolymer eines Monomers ist, das unter n-Butylmethacrylat, Cyclodecylacrylat, Neopentylacrylat und Isodecylacrylamid ausgewählt ist.

**28.** 28, Zusammensetzung nach einem der Ansprüche 1 bis 7 und 11 bis 26, **dadurch gekennzeichnet, dass** die Sequenz mit einer Tg zwischen 20 und 40 °C ein Copolymer, das ganz oder teilweise abgeleitet ist von:

- Monomeren, deren Homopolymer eine Tg von größer oder gleich 40 °C, insbesondere eine Tg im Bereich von 40 bis 150 °C, vorzugsweise von größer oder gleich 50 °C, insbesondere im Bereich von 50 bis 120 °C und bevorzugt von größer oder gleich 60 °C und insbesondere im Bereich von 60 bis 120 °C aufweist; und
- Monomeren, deren Homopolymer eine Tg von kleiner oder gleich 20 °C, insbesondere im Bereich von -100 bis 20 °C, vorzugsweise von 15 °C oder darunter, insbesondere im Bereich von -80 bis 15 °C und bevorzugt von 10 °C oder darunter, insbesondere im Bereich von -50 bis 0 °C aufweist.

**29.** Zusammensetzung nach einem der Ansprüche 1 bis 6 und 11 bis 26 und 28, **dadurch gekennzeichnet, dass** die Sequenz mit einer Tg zwischen 20 und 40°C ganz oder teilweise von Monomeren abgeleitet ist, die unter Methylmethacrylat, Isobornyl(meth)acrylat, Trifluorethylmethacrylat, Butylacrylat, 2-Ethylhexylacrylat und deren Gemischen ausgewählt sind.

**30.** Zusammensetzung nach einem der Ansprüche 1 bis 20 und 22 bis 24 und 26, 28, 29, **dadurch gekennzeichnet, dass** die erste Sequenz und/oder die zweite Sequenz mindestens ein ergänzendes Monomer enthalten.

**31.** Zusammensetzung nach dem vorhergehenden Anspruch, **dadurch gekennzeichnet, dass** das ergänzende Monomer unter den hydrophilen Monomeren, Monomeren mit ethylenisch ungesättigter Bindung, die ein oder mehrere Siliciumatome enthalten, und deren Gemischen ausgewählt ist.

**32.** Zusammensetzung nach Anspruch 30 oder 31, **dadurch gekennzeichnet, dass** das ergänzende Monomer ausgewählt ist unter:

Monomeren mit einer oder mehreren ethylenisch ungesättigten Bindungen, die mindestens eine Carbonsäurefunktion oder Sulfonsäurefunktion enthalten, Methacrylaten der Formel $CH_2 = C(CH_3)\text{-}COOR_6$,

wobei $R_6$ eine geradkettige oder verzweigte Alkylgruppe mit 1 bis 4 Kohlenstoffatomen bedeutet, wobei die Alkylgruppe mit einem oder mehreren Substituenten substituiert ist, die unter der Hydroxygruppe und den Halogenatomen ausgewählt ist;
Methacrylaten der Formel $CH_2 = C(CH_3)\text{-}COOR_9$,
wobei $R_9$ eine geradkettige oder verzweigte $C_{6-12}$-Alkylgruppe bedeutet, in deren Kette gegebenenfalls ein oder mehrere Heteroatome eingebaut sind, die unter O, N und S ausgewählt sind, wobei die Alkylgruppe mit einem oder mehreren Substituenten substituiert ist, die unter der Hydroxygruppe und den Halogenatomen ausgewählt ist;
Acrylaten der Formel $CH_2 = CHCOOR_{10}$, wobei $R_{10}$ eine geradkettige oder verzweigte $C_{1-12}$-Alkylgruppe bedeutet, die mit einem oder mehreren Substituenten substituiert ist, die unter der Hydroxygruppe und den Halogenatomen ausgewählt sind, oder $R_{10}$ Alkyl($C_{1-12}$)-O-POE (Polyoxyethylen) mit 5 bis 30 Oxyethylen-Wiederholungseinheiten bedeutet, oder $R_{10}$ eine Polyoxyethylengruppe mit 5 bis 30 Ethylenoxideinheiten ist, Monomeren mit einer oder mehreren ethylenisch ungesättigten Bindungen, die mindestens eine tertiäre Aminofunktion enthalten,
und deren Gemischen.

**33.** Zusammensetzung nach einem der Ansprüche 30 bis 32, **dadurch gekennzeichnet, dass** das oder die ergänzenden Monomere unter Acrylsäure, Methacrylsäure, Trifluorethylmethacrylat und deren Gemischen ausgewählt ist.

**34.** Zusammensetzung nach einem der Ansprüche 30 bis 33, **dadurch gekennzeichnet, dass** das oder die ergänzende (n) Monomer(e) 1 bis 30 Gew.-% des Gesamtgewichts der ersten und/ oder zweiten Sequenz ausmachen.

**35.** Zusammensetzung nach einem der vorhergehenden Ansprüche, **dadurch gekennzeichnet, dass** die erste und die zweite Sequenz jede mindestens ein Monomer enthalten, das unter den Estern der (Meth)acrylsäure ausgewählt

ist, und gegebenenfalls mindestens ein Monomer, das unter (Meth)acrylsäure ausgewählt ist, und deren Gemische.

**36.** Zusammensetzung nach einem der vorhergehenden Ansprüche, **dadurch gekennzeichnet, dass** die erste und die zweite Sequenz jede vollständig von mindestens einem Monomer, das unter den Estern der (Meth)acrylsäure ausgewählt ist, und gegebenenfalls mindestens einem Monomer, das unter (Meth)acrylsäure ausgewählt ist, und deren Gemischen abgeleitet ist.

**37.** Zusammensetzung nach einem der vorhergehenden Ansprüche, **dadurch gekennzeichnet, dass** die erste und die zweite Sequenz so sind, dass die Differenz der Glasübergangstemperaturen (Tg) der ersten und der zweiten Sequenz größer als 10 °C, besser größer als 20 °C, vorzugsweise größer als 30 °C und besonders bevorzugt größer als 40 °C ist.

**38.** Zusammensetzung nach einem der vorhergehenden Ansprüche, **dadurch gekennzeichnet, dass** die Zwischensequenz eine Glasübergangstemperatur aufweist, die zwischen den Glasübergangstemperaturen der ersten und der zweiten Sequenz liegt.

**39.** Zusammensetzung nach einem der vorhergehenden Ansprüche, **dadurch gekennzeichnet, dass** das Sequenzpolymer einen Polydispersitätsindex im Bereich von 2,8 bis 6 aufweist.

**40.** Zusammensetzung nach einem der vorhergehenden Ansprüche, **dadurch gekennzeichnet, dass** das Sequenzpolymer eine gewichtsmittlere Molmasse (Mw) von kleiner oder gleich 300 000, vorzugsweise im Bereich von 35 000 bis 200 000 und noch besser im Bereich von 45 000 bis 150 000 aufweist.

**41.** Zusammensetzung nach einem der vorhergehenden Ansprüche, **dadurch gekennzeichnet, dass** die zahlenmittlere Molmasse (Mn) kleiner oder gleich 70 000 ist, vorzugsweise im Bereich von 10 000 bis 60 000 und noch besser im Bereich von 12 000 bis 50 000 liegt.

**42.** Zusammensetzung nach einem der vorhergehenden Ansprüche, **dadurch gekennzeichnet, dass** das Polymer bei einem Gehalt der wirksamen Substanz von mindestens 1 Gew.-% in Wasser oder einem Gemisch von Wasser und linearen oder verzweigten, niederen Monoalkoholen mit 2 bis 5 Kohlenstoffatomen ohne pH-Wert-Änderung bei Raumtemperatur (25 °C) nicht löslich ist.

**43.** Zusammensetzung nach einem der vorhergehenden Ansprüche, **dadurch gekennzeichnet, dass** das Sequenzpolymer in einem Mengenanteil von 0,1 bis 90 Gew.-%, bezogen auf das Gesamtgewicht der Zusammensetzung, vorzugsweise 0,5 bis 50 Gew.-% und noch bevorzugter 0,5 bis 30 Gew.-% enthalten ist.

**44.** Zusammensetzung nach einem der vorhergehenden Ansprüche, **dadurch gekennzeichnet, dass** der Weichmacher eine Verbindung mit einem Solubilitätsparameter $\delta_h$ im Bereich von 5,5 bis 11 $(J/cm^3)^{1/2}$, vorzugsweise im Bereich von 5,5 bis 11 $(J/cm^3)^{1/2}$, bevorzugt im Bereich von 5,9 bis 11 $(J/cm^3)^{1/2}$, vorzugsweise im Bereich von 7 bis 10,5 $(J/cm^3)^{1/2}$ bevorzugt im Bereich von 9 bis 10 $(J/cm^3)^{1/2}$ und vorzugsweise im Bereich von 8 bis 10 $(J/cm^3)^{1/2}$ ist.

**45.** Zusammensetzung nach einem der vorhergehenden Ansprüche, **dadurch gekennzeichnet, dass** der Weichmacher eine Verbindung mit einem Solubilitätsparameter $\delta_p$ im Bereich von 1,5 bis 4,5 $(J/cm^3)^{1/2}$, vorzugsweise im Bereich von 1,5 bis 4 $(J/cm^3)^{1/2}$, vorzugsweise im Bereich von 1,5 bis 3,5 $(J/cm^3)^{1/2}$ und noch bevorzugter im Bereich von 2 bis 3 $(J/cm^3)^{1/2}$ ist.

**46.** Zusammensetzung nach einem der vorhergehenden Ansprüche, **dadurch gekennzeichnet, dass** der Weichmacher eine Molmasse von kleiner oder gleich 5000 g/mol, vorzugsweise von kleiner oder gleich 2000 g/mol, bevorzugt von kleiner oder gleich 1000 g/mol und besonders bevorzugt von kleiner oder gleich 900 g/mol aufweist, wobei er vorteilhaft eine Molmasse von größer oder gleich 100 g/mol besitzt.

**47.** Zusammensetzung nach einem der vorhergehenden Ansprüche, **dadurch gekennzeichnet, dass** der Weichmacher ein Ester ist.

**48.** Zusammensetzung nach einem der vorhergehenden Ansprüche, **dadurch gekennzeichnet, dass** der Weichmacher unter den Estern mindestens einer Carbonsäure mit 1 bis 7 Kohlenstoffatomen und eines Polyols mit mindestens 4 Hydroxygruppen ausgewählt ist.

**49.** Zusammensetzung nach dem vorhergehenden Anspruch, **dadurch gekennzeichnet, dass** das Polyol ein Zucker und vorzugsweise ein in Form eines Halbacetals cyclisierter Zucker ist.

**50.** Zusammensetzung nach Anspruch 48 oder 49, **dadurch gekennzeichnet, dass** das Polyol unter D-Ribose, D-Xylose, L-Arabinose, D-Glucose, D-Mannose, D-Galactose, D-Xylulose und D-Fructose ausgewählt ist.

**51.** Zusammensetzung nach Anspruch 50, **dadurch gekennzeichnet, dass** es sich bei dem Polyol um ein Mono- oder Polysaccharid handelt, das 1 bis 10 Zucker, vorzugsweise 1 bis 4 Zucker und noch bevorzugter ein oder zwei Zucker enthält.

**52.** Zusammensetzung nach Anspruch 48 oder 51, **dadurch gekennzeichnet, dass** das Polyol unter Erythrit, Xylit, Sorbit, Glucose, Saccharose, Lactose und Maltose ausgewählt ist.

**53.** Zusammensetzung nach einem der Ansprüche 48, 51, 52, **dadurch gekennzeichnet, dass** es sich bei dem Polyol um Saccharose handelt.

**54.** Zusammensetzung nach einem der Ansprüche 47 bis 52, **dadurch gekennzeichnet, dass** es sich bei dem Ester um ein mit mindestens zwei unterschiedlichen Monocarbonsäuren verestertes Polyol handelt.

**55.** Zusammensetzung nach einem der Ansprüche 47 bis 54, **dadurch gekennzeichnet, dass** die Carbonsäure eine Monocarbonsäure ist, die 1 bis 7 Kohlenstoffatome und vorzugsweise 1 bis 5 Kohlenstoffatome aufweist.

**56.** Zusammensetzung nach einem der Ansprüche 47 bis 55, **dadurch gekennzeichnet, dass** die Carbonsäure unter Essigsäure, n-Propansäure, Isopropansäure, n-Butansäure, Isobutansäure, tert-Butansäure, n-Pentansäure und Benzoesäure ausgewählt ist.

**57.** Zusammensetzung nach einem der vorhergehenden Ansprüche, **dadurch** gekenntzeichnet, dass der Weichmacher ein Saccharose-diacetat-hexa-(2-methyl-propanoat) ist.

**58.** Zusammensetzung nach einem der Ansprüche 1 bis 46, **dadurch gekennzeichnet, dass** der Weichmacher unter den Estern einer aliphatischen oder aromatischen Polycarbonsäure und eines aliphatischen oder aromatischen Alkohols mit 1 bis 10 Kohlenstoffatomen ausgewählt ist.

**59.** Zusammensetzung nach dem vorhergehenden Anspruch,
**dadurch gekennzeichnet, dass**:

- ■ der aliphatische Alkohol unter den Alkoholen R1OH, wobei R1 Methyl, Ethyl, Propyl, Isopropyl, Butyl, Hexyl, Ethylhexyl, Decyl, Isodecyl, Benzyl oder eine Benzylgruppe bedeutet, die mit einer Alkylgruppe mit 1 bis 3 Kohlenstoffatomen substituiert ist, und deren Gemischen ausgewählt ist;
- ■ die aliphatische oder aromatische Polycarbonsäure 3 bis 12 Kohlenstoffatome, vorzugsweise 3 bis 10 Kohlenstoffatome, bevorzugt 3 bis 8 Kohlenstoffatome und beispielsweise 6 oder 8 Kohlenstoffatome aufweist.

**60.** Zusammensetzung nach Anspruch 58 oder 59, **dadurch gekennzeichnet, dass** die aliphatische oder aromatische Polycarbonsäure unter den Dicarbonsäuren und Tricarbonsäuren ausgewählt ist.

**61.** Zusammensetzung nach einem der Ansprüche 58 bis 60, **dadurch gekennzeichnet, dass** die Polycarbonsäure eine Dicarbonsäure ist, die unter Bernsteinsäure, Adipinsäure, Sebacinsäure und Phthalsäure ausgewählt ist.

**62.** Zusammensetzung nach einem der Ansprüche 58 bis 60, **dadurch gekennzeichnet, dass** die Polycarbonsäure eine Säure der folgenden Formel ist:

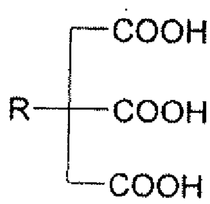

worin R eine Gruppe -H, -OH oder -OCOR' bedeutet, worin R' eine Alkylgruppe mit 1 bis 6 Kohlenstoffatomen bedeutet, wobei R vorzugsweise eine Gruppe $-OCOCH_3$ ist.

**63.** Zusammensetzung nach einem der Ansprüche 58 bis 60 und 62, **dadurch gekennzeichnet, dass** die Polycarbonsäure unter Acetylcitronensäure, Butyroyl-citronensäure und Citronensäure ausgewählt ist.

**64.** Zusammensetzung nach einem der Ansprüche 58 bis 63, **dadurch gekennzeichnet, dass** der Ester unter Tributyl-acetyl-citrat, Triethyl-acetyl-citrat, Triethylhexyl-acetyl-citrat, Trihexylacetyl-citrat, Trihexyl--butyroyl-citrat, Triisodecyl-citrat, Triisopropyl-citrat, Tributyl-citrat, Tri(2-ethylhexyl)-citrat, Dibutyladipat, Di(2-ethylhexyl)-adipat, Dibutylsebacat, Di(2-ethylhexyl)-sebacat, Diethylsebacat, Diisopropylsebacat, Di(2-ethylhexyl)-succinat, Diethylsuccinat, Butylbenzylphthalat, Dibutylphthalat, Diethylhexylphthalat, Diethylphthalat und Dimethylphthalat ausgewählt ist.

**65.** Zusammensetzung nach einem der Ansprüche 44 bis 64, **dadurch gekennzeichnet, dass** der Weichmacher mit Ausnahme der Estergruppe vorzugsweise keine polare Gruppe enthält und insbesondere keine Hydroxygruppe, wobei "polare" Gruppen ionische oder nichtionische polare Gruppen sind, die ausgewählt sind unter: -COOH; -OH; Ethylenoxid; Propylenoxid; $-PO_4$; -NHR; $-NR_1R_2$, wobei $R_1$ und $R_2$ eine Alkyl- oder Alkoxygruppe mit $C_1$ bis $C_{20}$ bedeuten, die linear, verzweigt oder cyclisch sein können.

**66.** Zusammensetzung nach einem der vorhergehenden Ansprüche, **dadurch gekennzeichnet, dass** der Weichmacher in einem Mengenanteil im Bereich von 0,1 bis 25 Gew.-%, bezogen auf das Gesamtgewicht der Zusammensetzung, vorzugsweise im Bereich von 0,5 bis 15 Gew.-% und bevorzugt im Bereich von 3 bis 15 Gew.-% enthalten ist.

**67.** Zusammensetzung nach einem der vorhergehenden Ansprüche, **dadurch gekennzeichnet, dass** das Sequenzpolymer und der Weichmacher in einer solchen Menge enthalten sind, dass das Masseverhältnis von Sequenzpolymer und Weichmacher im Bereich von 0,5 bis 100, vorzugsweise im Bereich von 1 bis 50, bevorzugt im Bereich von 1 bis 10 und besonders bevorzugt im Bereich von 1 bis 5 liegt.

**68.** Zusammensetzung nach einem der vorhergehenden Ansprüche, **dadurch gekennzeichnet, dass** sie ein flüchtiges Öl enthält.

**69.** Zusammensetzung nach einem der vorhergehenden Ansprüche, **dadurch gekennzeichnet, dass** sie ein flüchtiges Öl enthält, das unter Octamethylcyclotetrasiloxan, Decamethylcyclopentasiloxan, Dodecamethylcyclohexasiloxan, Heptamethylhexyltrisiloxan, Heptamethyloctyltrisiloxan, Octamethyltrisiloxan, Decamethyltetrasiloxan, Isododecan, Isodecan und Isohexadecan ausgewählt ist.

**70.** Zusammensetzung nach Anspruch 68 oder 69, **dadurch gekennzeichnet, dass** das flüchtige Öl in einem Mengenanteil von 1 bis 90 Gew.-%, bezogen auf das Gesamtgewicht der Zusammensetzung, vorzugsweise im Bereich von 1 bis 70 Gew.-% und noch bevorzugter im Bereich von 5 bis 50 Gew.-% enthalten ist.

**71.** Zusammensetzung nach einem der vorhergehenden Ansprüche, **dadurch gekennzeichnet, dass** sie ein nicht flüchtiges Öl enthält,

**72.** Zusammensetzung nach dem vorhergehenden Anspruch, **dadurch gekennzeichnet, dass** das nicht flüchtige Öl unter den nicht flüchtigen Kohlenwasserstoffölen und den nicht flüchtigen Siliconölen ausgewählt ist.

**73.** Zusammensetzung nach einem der vorhergehenden Ansprüche, **dadurch gekennzeichnet, dass** das nicht flüchtige Öl in einem Mengenanteil von 0,1 bis 20 Gew,-%, bezogen auf das Gesamtgewicht der Zusammensetzung, vorzugsweise im Bereich von 1 bis 15 Gew.-% und bevorzugt im Bereich von 1 bis 10 Gew.-% enthalten ist.

**74.** Zusammensetzung nach einem der vorhergehenden Ansprüche, **dadurch gekennzeichnet, dass** sie mindestens eine bei Raumtemperatur feste Fettsubstanz enthält, die unter den Wachsen, pastösen Fettsubstanzen, Gummis und deren Gemischen ausgewählt ist.

**75.** Zusammensetzung nach einem der vorhergehenden Ansprüche, **dadurch gekennzeichnet, dass** sie 0,1 bis 50 Gew.-% Wachse, bezogen auf das Gesamtgewicht der Zusammensetzung, und vorzugsweise 1 bis 30 Gew.-% enthält.

**76.** Zusammensetzung nach einem der vorhergehenden Ansprüche, **dadurch gekennzeichnet, dass** sie ein Farbmittel enthält.

**77.** Zusammensetzung nach einem der vorhergehenden Ansprüche, **dadurch gekennzeichnet, dass** sie einen kosmetischen Bestandteil enthält, der unter den ergänzenden filmbildenden Polymeren, Vitaminen, Verdickungsmitteln, Spurenelementen, beruhigenden Stoffen, Maskierungsmitteln, Parfums, Alkalisierungsmitteln oder Ansäuerungsmitteln, Konservierungsmitteln, Sonnenschutzfiltern, grenzflächenaktiven Stoffen, Antioxidantien, Wirkstoffen gegen Haarausfall, Antischuppenmitteln, Treibmitteln oder deren Gemischen ausgewählt ist.

**78.** Kosmetische Zusammensetzung nach einem der vorhergehenden Ansprüche, **dadurch gekennzeichnet, dass** sie als Suspension, Dispersion, Lösung, Gel, Emulsion und insbesondere Öl-in-Wasser-Emulsion (O/W) oder Wasser-in-Öl-Emulsion (W/O) oder multiple Emulsion (W/O/W oder Polyol/O/W oder O/W/O), als Creme, Paste, Schaum, Vesikeldispersion insbesondere von ionischen oder nichtionischen Lipiden, zweiphasige oder mehrphasige Lotion, Spray, Pulver, Paste, insbesondere weiche Paste oder wasserfreie Paste, vorliegt.

**79.** Kosmetische Zusammensetzung nach einem der vorhergehenden Ansprüche, **dadurch gekennzeichnet, dass** sie in wasserfreier Form vorliegt.

**80.** Kosmetische Zusammensetzung nach einem der vorhergehenden Ansprüche, **dadurch gekennzeichnet, dass** es sich um eine Zusammensetzung zum Schminken oder zur Pflege von Keratinsubstanzen handelt.

**81.** Zusammensetzung nach einem der vorhergehenden Ansprüche, **dadurch gekennzeichnet, dass** es sich um ein Produkt zum Schminken der Lippen handelt.

**82.** Kosmetische Einheit umfassend:

a) einen Behälter, der mindestens eine Abteilung abgrenzt,
wobei der Behälter mit einer Verschlusseinrichtung verschlossen ist, und
b) eine Zusammensetzung, die sich im Inneren der Abteilung befindet, wobei die Zusammensetzung einer Zusammensetzung nach einem der vorhergehenden Ansprüche entspricht.

**83.** Kosmetische Einheit nach Anspruch 82, **dadurch gekennzeichnet, dass** der Behälter zumindest zum Teil aus mindestens einem thermoplastischen Material gebildet ist.

**84.** Kosmetische Einheit nach Anspruch 82, **dadurch gekennzeichnet, dass** der Behälter zumindest zum Teil aus mindestens einem nichtthermoplastischen Material, insbesondere aus Glas oder Metall, gebildet ist.

**85.** Einheit nach einem der Ansprüche 82 bis 84, **dadurch gekennzeichnet, dass** in der geschlossenen Stellung des Behälters die Verschlusseinrichtung mit dem Behälter verschraubt ist.

**86.** Einheit nach einem der Ansprüche 82 bis 84, **dadurch gekennzeichnet, dass** in der geschlossenen Stellung des Behälters die Verschlusseinrichtung an den Behälter auf eine andere Weise als durch Verschrauben gekoppelt ist, insbesondere durch Einrasten, Kleben oder Schweißen.

**87.** Einheit nach einem der Ansprüche 82 bis 86, **dadurch gekennzeichnet, dass** die Zusammensetzung im Inneren der Abteilung in etwa unter Atmosphärendruck steht.

**88.** Einheit nach einem der Ansprüche 82 bis 86, **dadurch gekennzeichnet, dass** die Zusammensetzung im Inneren der Abteilung unter Druck steht.

**89.** Kosmetisches Verfahren zum Schminken oder für die Pflege von Keratinsubstanzen, das umfasst, eine kosmetische Zusammensetzung nach einem der Ansprüche 1 bis 81 auf die Keratinsubstanzen aufzutragen.

**90.** Verwendung einer Zusammensetzung nach einem der Ansprüche 1 bis 81, um auf den Keratinsubstanzen und insbesondere der Haut oder den Lippen eine Abscheidung und insbesondere eine Schminke zu erhalten, die weich und/oder lange angenehm ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6074654 A
- WO 02067877 A
- US 6513206 B
- GB 1324745 A
- WO 0026285 A
- FR 2798061 A
- JP 8109121 A, KAO
- US 4887622 A
- FR 2796529
- FR 2722380
- US 5492426 A
- FR 2761959
- WO 0103538 A
- FR 2806273
- FR 2775566
- FR 2727609
- WO 03018423 A
- FR 2791042
- FR 2792618


### Littérature non-brevet citée dans la description


- Polymer Handbook. John Wiley, 1989
- **C. M. HANSEN.** The three dimensional solubility parameters. *J. Paint Technol.,* 1967, vol. 39, 105
- **D.W. Van KREVELEN.** *Properties of polymers,* 1990, 190